# EUROPÄISCHE PATENTSCHRIFT

(11) **EP 0 541 946 B1**
(45) Veröffentlichungstag und Bekanntmachung des Hinweises auf die Patenterteilung: **17.07.1996**
(21) Anmeldenummer: 92116761.5
(22) Anmeldetag: 30.09.1992
(51) Int. Cl.: C07C 269/06, C07C 271/20, C07C 315/04, C07C 317/44, C07D 217/18, C07C 213/00

(54) **Verfahren zur diastereoselektiven reduktiven Pinakol-Kupplung von homochiralen alpha-Aminoaldehyden**
Process for diastereo selective reductive pinacol-coupling of homochiral alpha-aminoaldehydes
Procédé de couplage-pinacol réductant diastéréosélectif d'alpha-aminoaldéhydes

(30) Priorität: 07.10.1991 DE 4133202
(43) Veröffentlichungstag der Anmeldung: 19.05.1993
(73) Patentinhaber: HOECHST AKTIENGESELLSCHAFT, 65926 Frankfurt am Main (DE)
(72) Erfinder: Jacobi, Detlef, Dr., W-7302 Ostfildern (DE); Jendralla, Joachim-Heiner, Dr., W-6230 Frankfurt/Main 80 (DE); Kammermeier, Bernhard, Dr., W-6000 Frankfurt/Main 71 (DE)

(56) Entgegenhaltungen:
- EP-A- 0 402 646
- WO-A-91/18866
- DE-A- 4 030 350
- JOURNAL OF MEDICINAL CHEMISTRY Bd. 33, Nr. 10, 1990, WASHINGTON,D.C. Seiten 2687-2689 KEMPF D.J. ET AL 'Structure based C2 symmetric inhibitors of HIV protease'
- JOURNAL OF THE AMERICAN CHEMICAL SOCIETY Bd. 109, 1987, WASHINGTON,D.C. Seiten 6551 - 6553 PEDERSEN S.F. ET AL 'The first practical niobium(III) reagent in organic synthesis.'
- JOURNAL OF THE AMERICAN CHEMICAL SOCIETY Bd. 109, 1987, WASHINGTON,D.C. Seiten 3152 - 3154 PEDERSEN S.F. ET AL 'Convenient routes to vicinal diamines.'

## Beschreibung

Die Erfindung betrifft ein Verfahren zur Herstellung von optisch reinen symmetrischen Verbindungen der Formel I 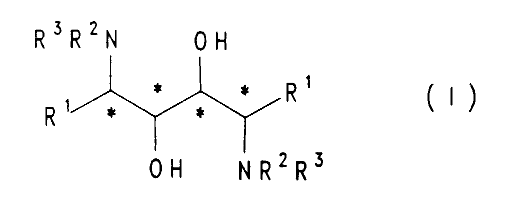
worin R¹, R² und R³ unten näher erläutert wird, unter gleichzeitiger Kontrolle der vier mit * gekennzeichneten Chiralitätszentren.

In EP-A 0402646 und D.J.Kempf et al. [J.Med.Chem. 33, 2687 (1990)] ist die Herstellung von Verbindungen obigen Typs unter Verwendung von McMurry-Reagenz [TiCl₃/Zn(Cu)] beschrieben. Diese reduktive Kupplungsmethode führt zu schwer trennbaren Gemischen. Wie in den oben zitierten Arbeiten berichtet, entstehen die drei Diastereomeren in etwa gleicher Menge und schlechter Ausbeute.

S.F.Pedersen et al. [J.Am.Chem.Soc. 109, 3152 (1987) und 109, 6551 (1987) beschrieben, daß sich mit den Niob (III)- bzw. Niob (IV)-Komplexen NbCl₃ (DME) und NbCl₄ (THF) bei der reduktiven Kreuzkupplung von achiralen Aldehyden mit achiralen Iminoaldehyden diastereoselektiv syn-Aminoaldehyde synthetisieren lassen, bzw. diastereoselektive syn-Diamine bei der reduktiven Kupplung von Iminoaldehyden entstehen.

Ziel der vorliegenden Erfindung ist es, ein einfacheres und stereoselektives Verfahren zur Herstellung der obengenannten Verbindungen (I) zu finden, welches die bekannten Nachteile nicht besitzt.

Die Erfindung wird gelöst durch das Verfahren zur Herstellung von Verbindungen der Formel I 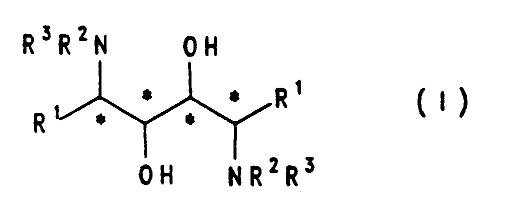
worin
- R¹: für eine Seitenkettenrest einer natürlichen oder nicht-natürlichen α-Aminosäure steht;
- R² und R³: gleich oder verschieden sind und
a)
   - Wasserstoff
b)
   - einen Rest der Formel

      D-(E)ₙ-(F)ₒ-(G)ₚ (II)

      bedeuten, wobei E, F und G unabhängig voneinander für eine natürliche oder unnatürliche Aminosäure, Azaaminosäure oder Iminosäure stehen;
      n, o, p unabhängig voneinander 0 oder 1 bedeuten;
      D für R⁴ oder einen Rest der Formeln III, IV oder V steht 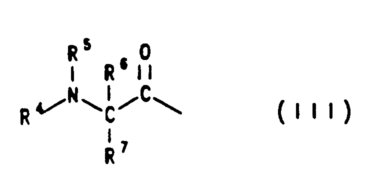 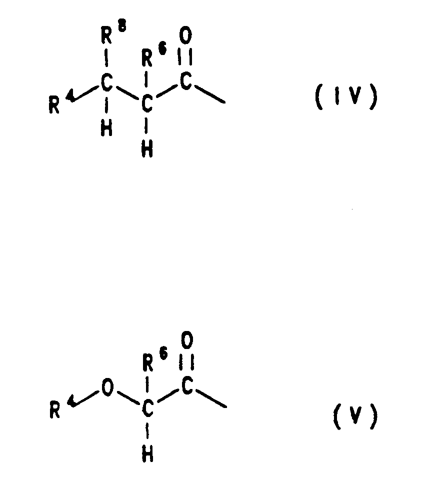 worin R⁴ bedeutet
b₁)
   - Wasserstoff,
   - Carboxyl,
   - (C₁-C₁₈)-Alkyl, das gegebenenfalls einfach oder zweifach ungesättigt ist und das gegebenenfalls durch bis zu 3 gleiche oder verschiedene Reste aus der Reihe
      - Mercapto,
      - Hydroxy,
      - (C₁-C₇)-Alkoxy,
      - Carbamoyl,
      - (C₁-C₈)-Alkanoyloxy,
      - Carboxy,
      - (C₁-C₇)-Alkoxycarbonyl,
      - F, Cl, Br, I,
      - Amino,
      - Amidino, das gegebenenfalls durch einen, zwei oder drei (C₁-C₈)-Alkylreste substituiert sein kann,
      - Guanidino, das gegebenenfalls durch einen oder zwei Benzyloxycarbonylreste oder durch einen, zwei, drei oder vier (C₁-C₈)-Alkylreste substituiert sein kann,
      - (C₁-C₇)-Alkylamino,
      - Di-(C₁-C₇)-Alkylamino,
      - (C₁-C₆)-Alkoxycarbonylamino,
      - (C₇-C₁₅)-Aralkoxycarbonyl,
      - (C₇-C₁₅)-Aralkoxycarbonylamino,
      - Phenyl-(C₁-C₄)-alkoxy,
      - 9-Fluorenylmethoxycarbonylamino,
      - (C₁-C₆)-Alkysulfonyl,
      - (C₁-C₆)-Alkylsulfinyl,
      - (C₁-C₆)-Alkylthio,
      - Hydroxamino,
      - Hydroximino,
      - Sulfamoyl,
      - Sulfo,
      - Carboxamido,
      - Formyl,
      - Hydrazono,
      - Imino,
      - einen Rest CONR⁹R¹⁰,
      - durch bis zu sechs Hydroxy oder
      - durch bis zu fünf (C₁-C₈)-Alkanoyloxy substituiert ist;
   - mono-, bi- oder tricyclisches (C₃-C₁₈)-Cycloalkyl,
   - (C₃-C₁₈)-Cycloalkyl-(C₁-C₆)-alkyl,
      wobei der Cycloalkylteil jeweils gegebenenfalls durch einen oder zwei gleiche oder verschiedene Reste aus der Reihe
      - F, Cl, Br, I,
      - Carboxy,
      - Carbamoyl,
      - Carboxymethoxy,
      - Hydroxy,
      - (C₁-C₇)-Alkoxy,
      - (C₁-C₇)-Alkyl,
      - (C₁-C₇)-Alkyloxycarbonyl,
      - Amino,
      - (C₁-C₆)-Alkylamino-(C₁-C₆)-alkyl,
      - Di-(C₁-C₆)-Alkylamino-(C₁-C₆)-alkyl,
      - Amidino,
      - Hydroxamino,
      - Hydroximino,
      - Hydrazono,
      - Imino,
      - Guanidino,
      - (C₁-C₆)-Alkoxysulfonyl,
      - (C₁-C₆)-Alkoxysulfinyl,
      - (C₁-C₆)-Alkoxycarbonylamino,
      - (C₆-C₁₂)-Aryl-(C₁-C₄)-alkoxycarbonylamino,
      - (C₁-C₇)-Alkylamino,
      - Di-(C₁-C₇)-alkylamino und
      - Trifluormethyl substituiert ist;
   - (C₆-C₁₄)-Aryl,
   - (C₆-C₁₄)-Aryl-(C₁-C₆)-alkyl,
   - (C₆-C₁₄)-Aryloxy-(C₁-C₆)-alkyl oder
   - (C₆-C₁₄)-Aryl-(C₃-C₈)-cycloalkyl,
      worin der Arylteil jeweils gegebenenfalls durch einen, zwei oder drei gleiche oder verschiedene Reste aus der Reihe
      - F, Cl, Br, I,
      - Hydroxy,
      - Mono-, Di- oder Trihydroxy-(C₁-C₄)-alkyl,
      - Trifluormethyl,
      - Formyl,
      - Carboxamido,
      - Mono- oder Di- (C₁-C₄)-alkylaminocarbonyl,
      - Nitro,
      - (C₁-C₇)-Alkoxy,
      - (C₁-C₇)-Alkyl,
      - (C₁-C₇)-Alkoxycarbonyl,
      - Amino,
      - (C₁-C₇)-Alkylamino,
      - Di-(C₁-C₇)-alkylamino,
      - Carboxy,
      - Carboxymethoxy,
      - Amino-(C₁-C₇)-alkyl,
      - (C₁-C₇)-Alkylamino-(C₁-C₇)-alkyl,
      - Di-(C₁-C₇)-alkylamino-(C₁-C₇)-alkyl,
      - (C₁-C₇)-Alkoxycarbonylmethoxy,
      - Carbamoyl,
      - Sulfamoyl,
      - (C₁-C₇)-Alkoxysulfonyl,
      - (C₁-C₈)-Alkylsulfonyl,
      - Sulfo-(C₁-C₈)-alkyl,
      - Guanidino-(C₁-C₈)-alkyl und
      - (C₁-C₆)-Alkoxycarbonylamino substituiert ist;
   - Het,
   - Het-(C₁-C₆)-alkyl,
   - Het-(C₃-C₈)-cycloalkyl,
   - Het-(C₃-C₈)-cycloalkyl-(C₁-C₄)-alkyl,
   - Het-(C₃-C₈)-cycloalkoxy-(C₁-C₄)-alkyl,
   - Het-thio-(C₁-C₆)-alkyl,
   - Het-thio-(C₃-C₈)-cycloalkyl,
   - Het-thio-(C₃-C₈)-cycloalkyl-(C₁-C₄)-alkyl,
      wobei Het jeweils für den Rest eines 5- bis 7-gliedrigen monocyclischen oder 8- bis 10-gliedrigen bicyclischen Ringsystems steht, das benzanelliert, aromatisch, teil-oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter b₁) definiert und/oder mit Oxo mono-, di- oder trisubstituiert ist, oder einen Rest NR⁹R¹⁰ bedeutet, oder
b₂)
   - einen Rest der Formel VI

      R^{4a} - W (VI)

      worin R^{4a} wie R⁴ unter b₁) definiert ist und W für -CO-, -CS-, -O-CO-, -SO₂-, -SO-, -S-, -NHSO₂-, -NHCO-, -CH(OH)-, -N(OH)-oder -CO-V-, wobei V ein Peptid mit insgesamt 1 bis 10 Amino-, Imino- und/oder Azaaminosäuren bedeutet, steht;
      oder worin R⁴ zusammen mit R⁸ und den diese tragenden Atomen mono-oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
b₃)
   - einen Glycosylrest, bevorzugt einen Glucofuranosyl-oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet; oder
b₄)
   - eine Aminoschutzgruppe;
- R⁵: - Wasserstoff oder
- (C₁-C₈)-Alkyl bedeutet, oder
- zusammen mit R⁶ und den diesen Rest tragenden Atomen mono-oder bicyclische, gesättigte oder teilweise ungesättigte Ringsysteme mit 5-12 Ringgliedern bildet;
- R⁶: - wie R⁴ unter b₁) definiert ist;
- für Hydroxy oder (C₁-C₄)-Alkanoyloxy steht; oder
- zusammen mit R⁷ und den diesen Rest tragenden Atomen cyclische, gesättigte oder teilweise ungesättigte Ringsysteme mit 3 bis 12 Ringgliedern bildet; oder
- zusammen mit R⁸ und den diese tragenden Atomen ein mono-oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
- R⁷: - Wasserstoff oder
- (C₁-C₆)-Alkyl bedeutet;
- R⁸: - Wasserstoff,
- Hydroxy,
- (C₁-C₄)-Alkanoyloxy oder
- (C₁-C₈)-Alkyl bedeutet;
- R⁹ und R¹⁰: - Wasserstoff,
- (C₁-C₈)-Alkyl, das durch
   - Amino,
   - (C₁-C₄)-Alkylamino,
   - Di-(C₁-C₄)-alkylamino,
   - Mercapto,
   - Carboxy,
   - Hydroxy oder
   - (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-alkoxycarbonyl, die im Arylteil wie bei R⁴ beschrieben, substituiert sein können,
- Het oder
- Het-(C₁-C₄)-alkyl, wobei Het wie bei R⁴ beschrieben definiert ist, bedeuten oder wobei
R⁹ und R¹⁰ zusammen mit dem sie tragenden Stickstoffatom monocyclische oder bicyclische, gesättigte, teilweise ungesättigte oder aromatische Ringsysteme bilden, die als Ringglieder neben Kohlenstoff noch 1 oder weitere 2 Stickstoffatome, 1 Schwefelatom oder 1 Sauerstoffatom enthaften und durch (C₁-C₄)-Alkyl substituiert sein können, wobei in den vorstehenden Verbindungen der Formel I eine oder mehrere Amidgruppen (-CONH-) der Hauptkette ersetzt sein können durch -CH₂NR¹¹-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹²)CH₂- und -P(O)(OR¹²)NH-, oder auch durch eine Amidgruppe mit umgekehrter Polarität (-NHCO-);
worin R¹¹ und R¹² unabhängig voneinander stehen für
- Wasserstoff oder
- (C₁-C₄)-Alkyl;
sowie deren Enantiomere und physiologisch verträgliche Salze, dadurch gekennzeichnet, daß homochirale α-Aminoaldehyde der Formel VII 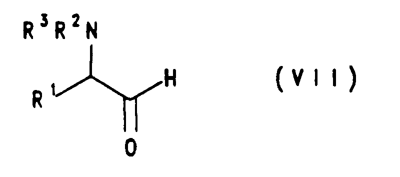
worin R¹, R² und R³ wie oben definiert sind, mit dem NbCl₃-Dimethoxyethan-Komplex [NbCl₃ (DME)] behandelt werden, wobei eine gleichzeitige Kontrolle über alle vier Chiralitätszentren vorliegt. Bevorzugt werden Verbindungen der Formel I hergestellt, worin
- R¹: einen Seitenkettenrest der α-Aminosäuren Gly, Ala, Val, Leu, Ile, Ser, Thr, Cys, Met, Pro, Lys, Arg, His, Asp, Asn, Glu, Gln, Phe, Tyr, Trp oder Cha bedeutet;
- R² und R³: gleich oder verschieden sind und
a)
   - Wasserstoff
b)
   - einen Rest der Formel II
      worin o und p = 0,
      n = 0 oder 1 ist und
      E für eine der oben genannten α-Aminosäuren steht,
      D für R⁴ oder einen Rest der Formeln III oder IV steht, worin R⁴
b₁)
   - Wasserstoff
   - (C₁-C₉)-Alkyl, das gegebenenfalls einfach oder zweifach ungesättigt ist und das gegebenenfalls durch bis zu 3 gleiche oder verschiedene Reste aus der Reihe
      - Hydroxy,
      - (C₁-C₇)-Alkoxy,
      - Carbamoyl,
      - (C₁-C₈)-Alkanoyloxy,
      - (C₁-C₇)-Alkoxycarbonyl,
      - F, Cl,
      - Amino,
      - (C₁-C₇)-Alkylamino,
      - Di-(C₁-C₇)-Alkylamino,
      - (C₁-C₆)-Alkoxycarbonylamino,
      - (C₇-C₁₅)-Aralkoxycarbonyl,
      - (C₇-C₁₅)-Aralkoxycarbonylamino,
      - Phenyl-(C₁-C₄)-alkoxy,
      - 9-Fluorenylmethoxycarbonylamino,
      - (C₁-C₆)-Alkylsulfonyl,
      - (C₁-C₆)-Alkylsulfinyl,
      - (C₁-C₆)-Alkylthio subsitutiert ist,
   - (C₆-C₁₄)-Aryl,
   - (C₆-C₁₄)-Aryl-(C₁-C₆)-alkyl oder
   - (C₆-C₁₄)-Aryloxy-(C₁-C₆)-alkyl, worin der Arylteil jeweils gegebenenfalls durch einen, zwei oder drei gleiche oder verschiedene Reste aus der Reihe der oben genannten bevorzugten Substituenten von (C₁-C₉)-Alkyl substituiert sein kann,
b₂)
   - einen Rest der Formel VI, worin
      R^{4a} wie R⁴ unter b₁) definiert ist und W für - CO-, O-CO-, -SO₂-, -SO-, -S-, -NHCO-, -CH(OH)- steht;
b₄)
   - eine Aminoschutzgruppe Fmoc, Z oder Boc bedeutet,
- R⁵ und R⁷: Wasserstoff bedeuten,
- R⁶: - wie R⁴ definiert ist, und
- R⁸: - Wasserstoff,
- Hydroxy,
- (C₁-C₄)-Alkanoyloxy oder
- (C₁-C₈)-Alkyl bedeutet.

Bevorzugt sind ferner Verbindungen der Formel I, worin einer der Reste R² oder R³ Wasserstoff bedeutet.

Weiterhin sind Verbindungen der Formel I mit SRRS-Konfiguration (wenn Aldehyde der Formel VII mit (S)-Konfiguration eingesetzt werden) bzw. Verbindungen der Formel I mit der RSSR-Konfiguration (wenn Aldehyde der Formel VII mit (R)-Konfiguration eingesetzt werden) bevorzugt.

Ganz besonders bevorzugt sind Verbindungen der Formel I, worin
- R¹: - einen Seitenkettenrest der α-Aminosäuren Ala, Val, Leu, Ile, Pro, Phe, Cha oder Tyr bedeutet,
- R² und R³: gleich oder verschieden sind und
a)
   - Wasserstoff
b)
   - einen Rest der Formel II, worin
      o und p = 0 sind,
      n 0 oder 1 bedeutet und
      E für Ala, Val, Leu, Ile, Pro, Phe, Cha oder Tyr steht;
      D für R⁴ oder einen Rest der Formel IV steht mit R⁴
b₁)
   - Wasserstoff,
   - (C₁-C₄)-Alkyl,
   - Phenyl oder Naphthyl,
   - Phenylmethyl oder Naphthylmethyl,
b₂)
   - Rest der Formel VI, worin R^{4a} wie R⁴ unter b₁) definiert ist und W für -CO-, -O-CO-, -SO₂-, -SO-, -S-, -NHCO-, -CH(OH)- steht, oder
b₄)
   - eine Aminoschutzgruppe Fmoc, Z oder Boc bedeuten,
- R⁵, R⁷ und R⁸: Wasserstoff bedeuten, und
- R⁶: wie R⁴ unter b₁) definiert ist.

Weitere bevorzugte Verbindungen sind die Beispielverbindungen der Europäischen Patentanmeldung 0 428 849.

Ganz besonders bevorzugt sind weiterhin Verbindungen der Formel I mit der SRRS-Konfiguration, erhalten unter Einsatz von Aldehyden der Formel VII mit (S)-Absolutkonfiguration. Diese Aussage gilt nur unter der Voraussetzung, daß die Gruppe R¹ niedrigere Cahn-Ingold-Prolog-Priorität als die Gruppe -CH(OH)-CH(OH)- hat.

α-Aminosäuren können, falls chiral, in der S- oder R-Form vorliegen. Sie entsprechen nachstehender Formel VIII 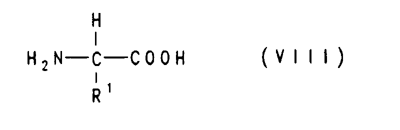
und unterscheiden sich lediglich im Rest R¹ der Seitenkette. Beispielhaft sind nachstehend einige natürliche und nicht-natürliche α-Aminosäuren im Dreibuchstabencode genannt: 
Aad, Abu, ABz, 2ABz, Ach, Acp, Adpd, Ahb, Aib, Ala, Alg, All, Ama, Amt, Ape,
Apm, Apr, Arg, Asn, Asp, Asu, Aze, Azi, Bai, Bph, Can, Cha, Cit, Cys, (Cys)2, Cyta,
Daad, Dab, Dadd, Dap, Dapm, Dasu, Djen, Dpa, Dtc, Fel, Gln, Glu, Gly, Guv, hAla,
hArg, hCys, hGln, hGlu, His, hIle, hLeu, hLys, hMet, hPhe, hPro, hSer, hThr, hTrp,
Hyl, Hyp, 3Hyp, Ile, Ise, Iva, Kyn, Lant, Lcn, Leu, Lsg, Lys, Met, Mim, Min, nArg,
Nle, Nva, Oly, Orn, Pan, Pec, Pen, Phe, Phg, Pic, Pro, Pse, Pya, Pyr, Pza, Qin,
Ros, Sar, Sec, Sem, Ser, Thi, Thr, Thy, Thx, Tia, Tle, Tly, Trp, Trta, Tyr, Val, Nal,
Tbg, Npg, Chg, Thia, Cha
(siehe z.B. Houben-Weyl, "Methoden der organischen Chemie" Band XV/1 und 2, Stuttgart 1974). Falls in Einzelverbindungen nicht anders angegeben, steht die Abkürzung eines Aminosäurerestes ohne einen Stereodeskriptor für den Rest in der L-Form, welche üblichenweise der S-Konfiguration entspricht.

Unter einer Iminosäure werden allgemein natürliche oder unnatürliche Aminosäuren verstanden, deren Aminogruppe monosubstituiert ist. Besonders seien in diesem Zusammenhang Verbindungen genannt, die durch (C₁-C₈)-Alkyl substituiert sind. Ferner kommen Heterocyclen aus der folgenden Gruppe in Betracht:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro[(bicyclo[2.2.1]heptan)-2,3-pyrrolidin-5-carbonsäure]; Spiro[(bicyclo[2.2.2]octan)-2,3-pyrrolidin-5-carbonsäure]; 2-Azatricyclo[4.3.0.1^{6,9}]decan-3-carbonsäure; Decahydrocyclohepta[b]pyrrol-2-carbonsäure; Decahydrocycloocta[b]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure, die alle gegebenenfalls substituiert sein können:

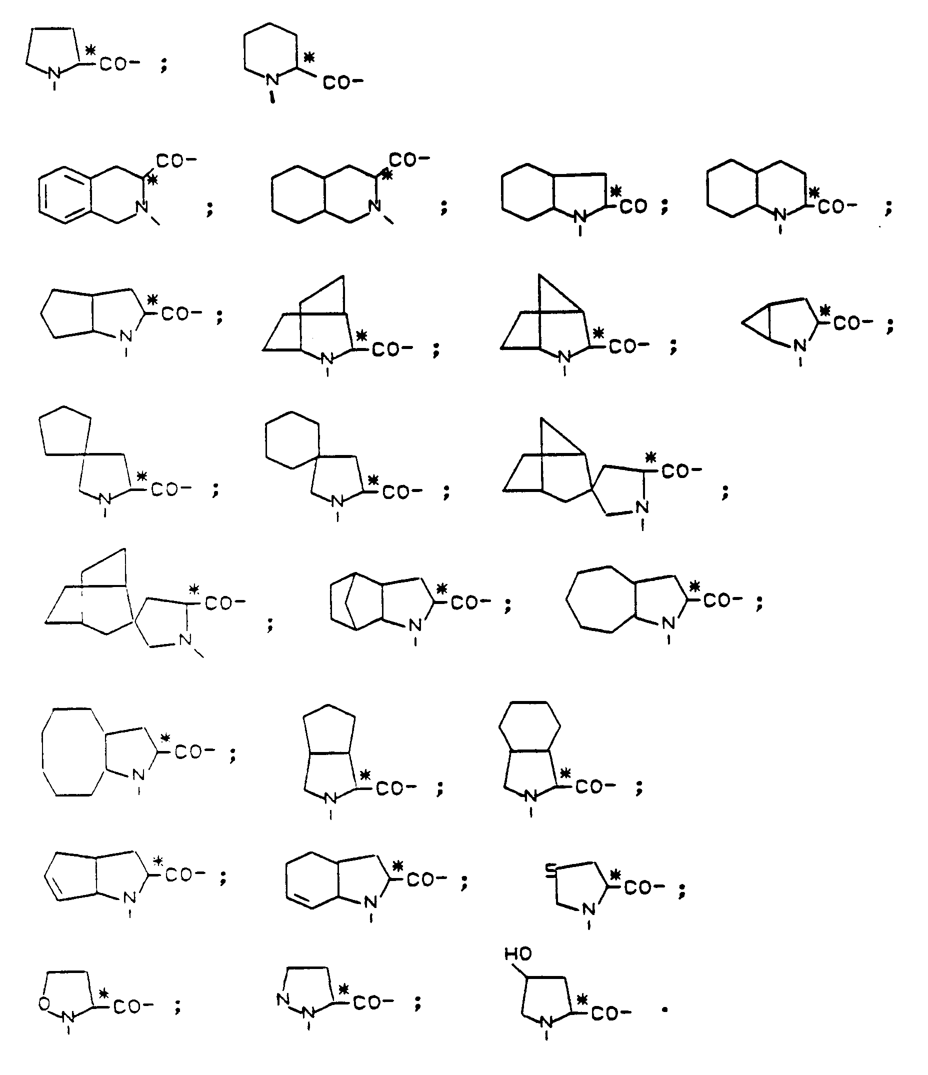

Azaaminosäuren sind von natürlichen oder nicht-natürlichen Aminosäuren abgeleitet, wobei der Zentralbaustein -CHR- bzw. -CH₂- durch -NR- bzw. - NH- ersetzt ist.

Einen Überblick über die Synthesen, insbesondere der nicht-natürlichen optisch aktiven α-Amino- und Iminosäuren gibt R.M. Williams in "Synthesis of Optically Active α-Aminoacids", Pergamon Press, Oxford 1989.

Die in dieser Beschreibung benutzte Nomenklatur folgt der allgemeinen Praxis bei Aminosäuren, das heißt, die Aminosäure steht links, die Carboxygruppe rechts von jeder Aminosäure. Entsprechendes gilt für Iminosäuren und Azaaminosäuren.

Aminoschutrgruppen sind in R.Geiger und W.König "The Peptides" Volume 3 "Protection of Functional Groups in Peptide Synthesis", E.G.Gross, J.Meienhofer Edit., Academic Press, New York (1981), insbesondere Seiten 7 - 46, beschrieben. Einige sind nachfolgend beispielhaft angegeben:

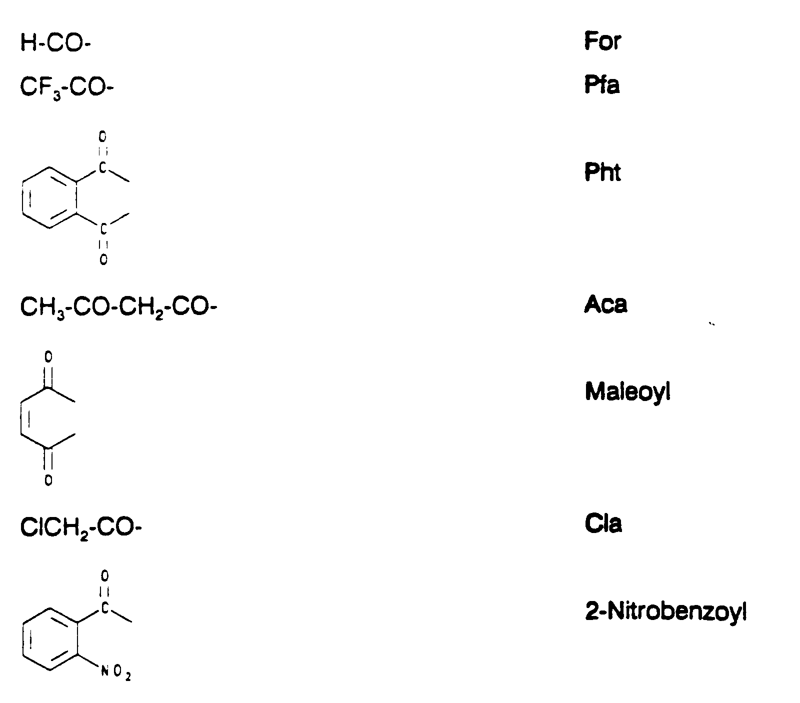

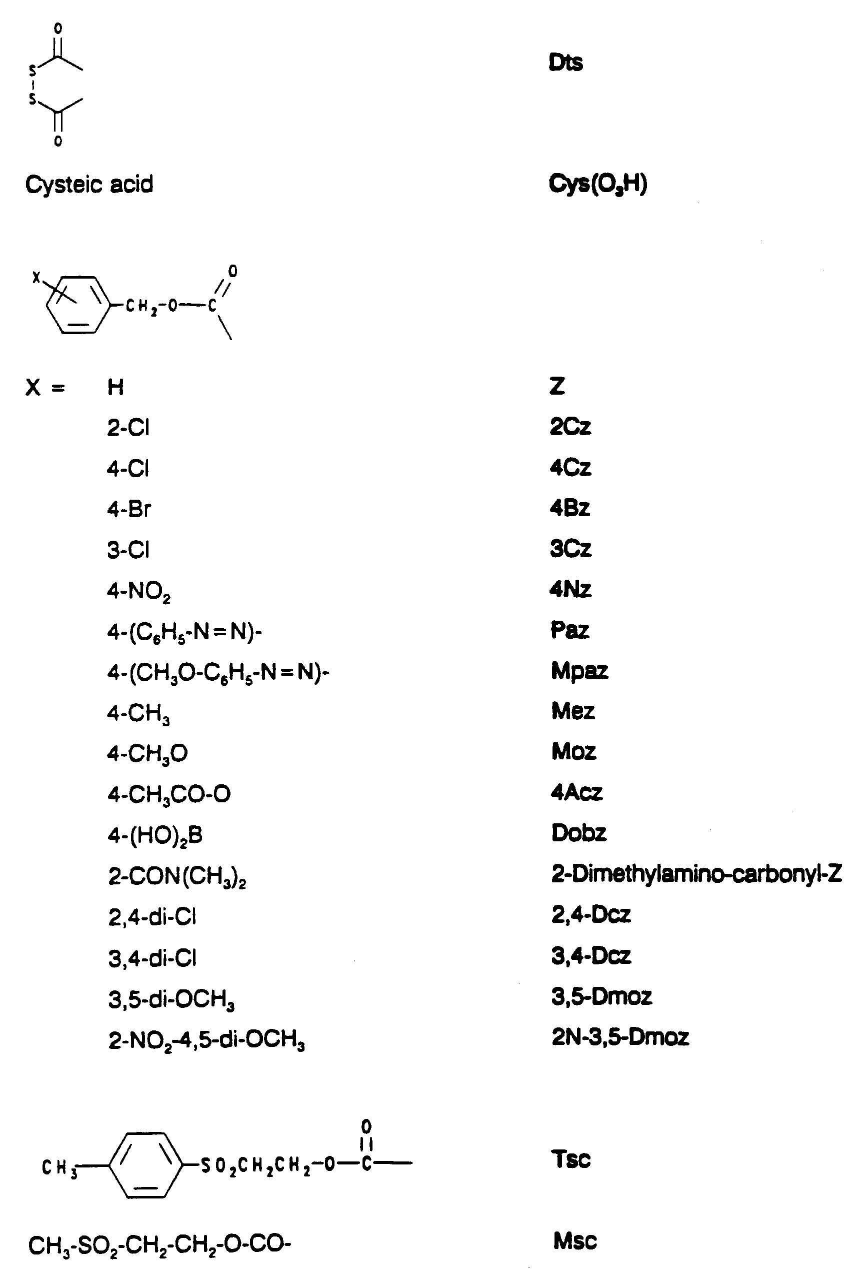

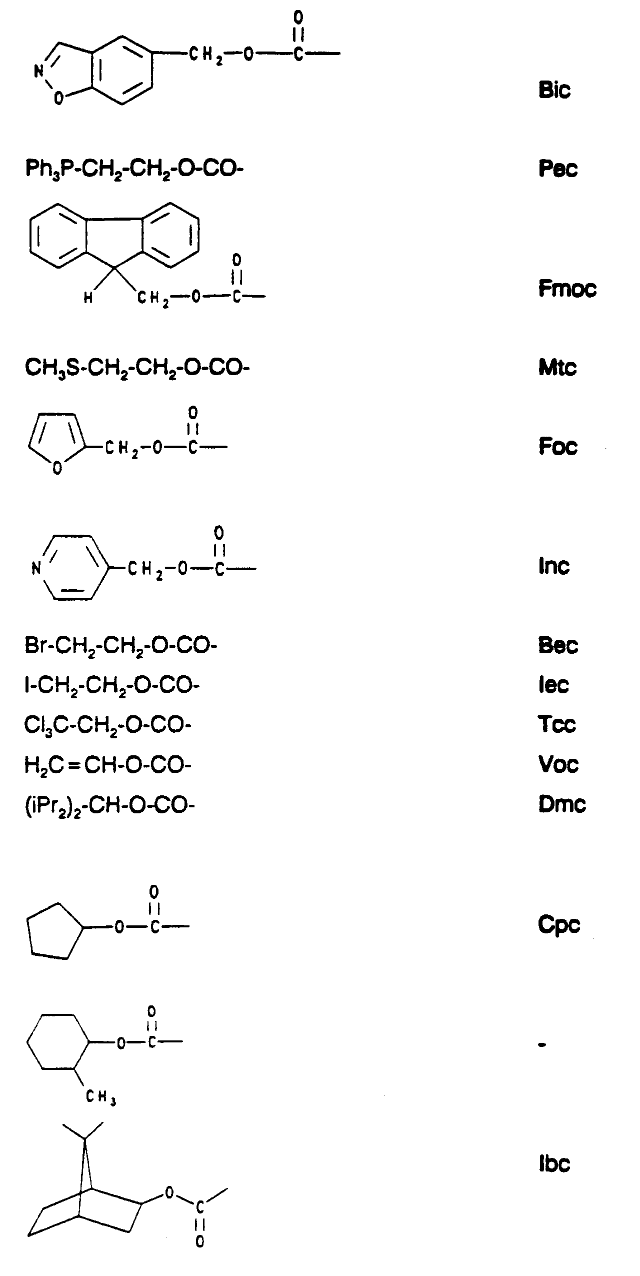

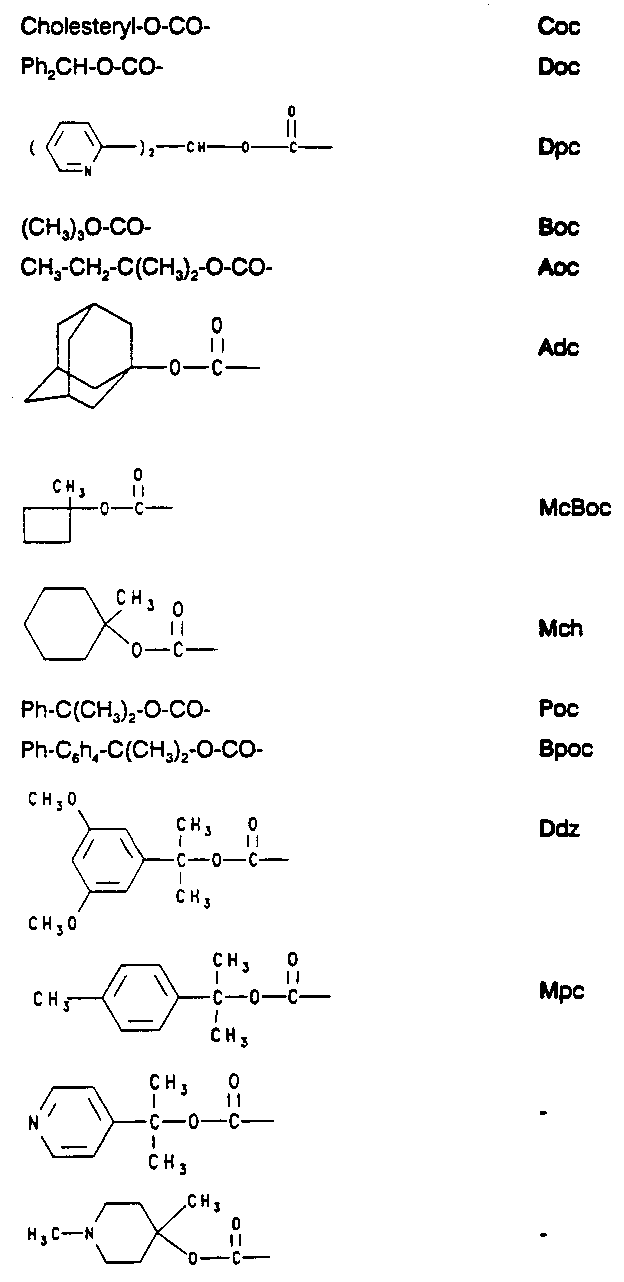

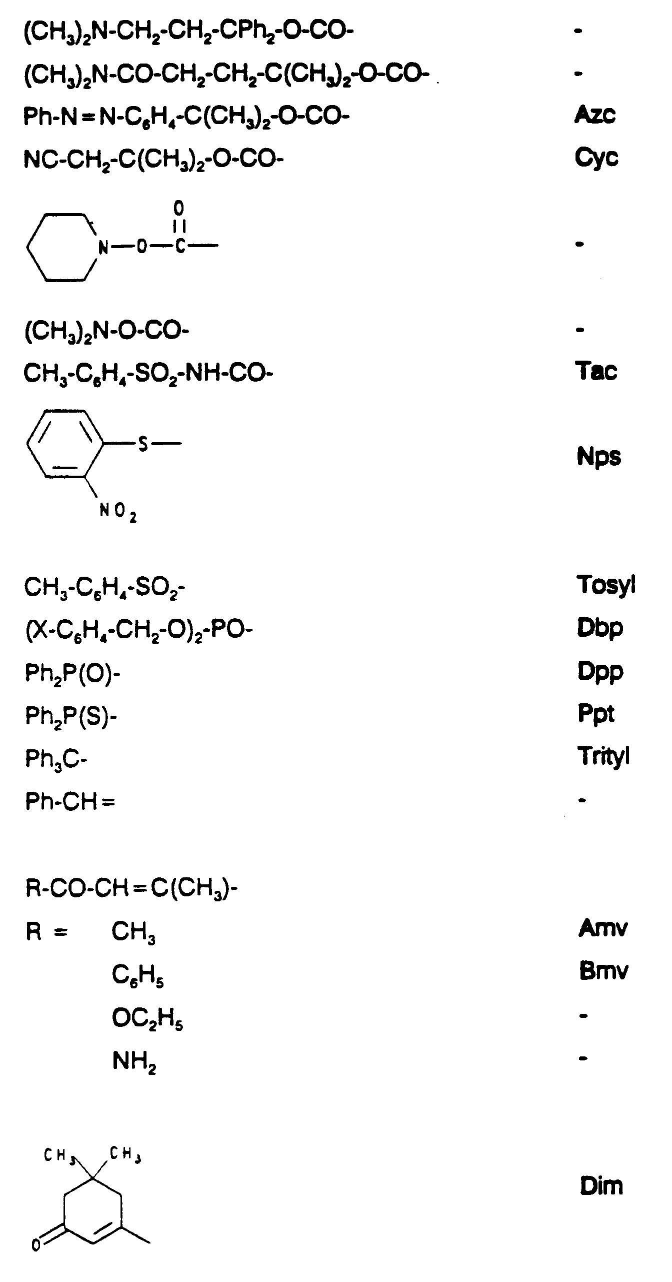

Funktionelle Gruppen in den Seitenketten der Amino-, Imino- oder Azaaminosäuren können beispielsweise wie folgt geschützt werden:
a) die Guanidinogruppe (z.B. von Arginin) kann gemäß Geiger/König in E. Gross, J. Meienhofer ("The Peptides- Protection of Functional Groups in Peptide Synthesis", Academic Press, New York, 1981), S. 60 - 70 geschützt werden;
b) der Aminostickstoff (z.B. von Lysin) kann gemäß S. 7 - 49 geschützt werden;
c) der Imidazolstickstoff (z.B. von Histidin) kann gemäß S. 70 - 80 geschützt werden;
d) der Pyrazolylstickstoff (z.B. von β-3-Pyrazolylalanin) kann gemäß S. 81 - 82 geschützt werden;
e) der Indolstickstoff (z.B. von Tryptophan) kann gemäß S. 82 - 84 geschützt werden;
f) die Carboxylgruppe (z.B. von Asparaginsäure) kann gemäß S. 102 - 132 geschützt werden;
g) die Sulfhydrylgruppe (z.B. von Cystein) kann gemäß S. 137 - 169 geschützt werden;
h) die Hydroxylgruppe (z.B. von Serin, Threonin, Tyrosin) kann gemäß S. 170 - 201 geschützt werden;
i) im Falle, daß R² einer Peptidgruppe entspricht, können, wo erforderlich, peptidische Amidstickstoffe gemäß S. 52-59 geschützt werden.

Glycosylreste wie vorstehend beschrieben leiten sich insbesondere von natürlichen, in Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacohariden, wie Maltose (Mal), Lactose (Lac); Cellobiose (Cel), Gentibiose (Gen), N-Acetyl-lactosamin (LacNAc), Chitobiose (Chit), β-Galactopyranosyl-(1-3)-N-acetylgalactosamin und β-Galactopyranosyl-(1-3)- oder (1-4)-N-acetyl-glucosamin, sowie deren synthetischen Derivaten, wie 2-Desoxy-, 2-Amino-, 2-Acetamido- oder 2-Halogeno-, bevorzugt Bromo- und Jodo-Zucker ab.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

(C₆-C₁₄)-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkoxy. Unter Aralkyl versteht man einen mit (C₁-C₆)-Alkyl verknüpften unsubstituierten oder substituierten (C₆-C₁₄)-Aryl-Rest, wie z.B. Benzyl, 1- und 2-Naphthylmethyl, wobei Aralkyl jedoch nicht auf die genannten Reste beschränkt wäre.

Reste Het im Sinne vorstehender Definition sind Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benzanelliertes, cyclopenta-, cyclohexa- oder cyclohepta-anelliertes Derivat dieser Reste.

Diese Heterocyclen können an einem Stickstoffatom durch Oxide; (C₁-C₇)-Alkyl, z.B. Methyl oder Ethyl; Phenyl; Phenyl-(C₁-C₄)-alkyl, z.B. Benzyl; und/oder an einem oder mehreren Kohlenstoffatomen durch (C₁-C₄)-Alkyl, z.B. Methyl; Phenyl; Phenyl-(C₁-C₄)-alkyl, z.B. Benzyl; Halogen; Hydroxy; (C₁-C₄)-Alkoxy, z.B. Methoxy; Phenyl-(C₁-C₄)-alkoxy, z.B. Benzyloxy; oder Oxo substituiert und teilweise oder vollständig gesättigt sein.

Derartige Reste sind beispielsweise 2- oder 3-Pyrrolyl; Phenyl-pyrrolyl, z.B. 4- oder 5-Phenyl-2-pyrrolyl; 2-Furyl; 2-Thienyl; 4-Imidazolyl; Methyl-imidazolyl, z.B. 1-Methyl-2-, -4- oder -5-imidazolyl; 1,3-Thiazol-2-yl; 2-, 3- oder 4-Pyridyl; 2-, 3- oder 4-Pyridyl-N-oxid; 2-Pyrazinyl; 2-, 4- oder 5-Pyrimidinyl; 2-, 3- oder 5-Indolyl; substituiertes 2-Indolyl, z.B. 1-Methyl-, 5-Methyl-, 5-Methoxy-, 5-Benzyloxy-, 5-Chlor- oder 4,5-Dimethyl-2-indolyl; 1- Benzyl- 2- oder -3-indolyl; 4,5,6,7-Tetrahydro-2-indolyl; Cyclohepta[b]-5-pyrrolyl; 2-, 3- oder 4-Chinolyl; 1-, 3- oder 4-Isochinolyl; 1-Oxo-1,2-dihydro-3-isochinolyl, 2-Chinoxalinyl; 2-Benzofuranyl; 2-Benzoxazolyl; Benzothiazolyl; Benz[e]indol-2-yl oder β-Carbolin-3-yl.

Teilhydrierte oder vollständig hydrierte heterocyclische Ringe sind beispielsweise Dihydropyridinyl; Pyrrolidinyl, z.B. 2-, 3- oder 4-N-Methylpyrrolidinyl; Piperazinyl; Morpholino; Thiomorpholino; Tetrahydrothiophenyl; Benzodioxolanyl.

Halogen steht für Fluor, Chlor, Brom oder Jod, insbesondere für Fluor oder Chlor.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin.

Verbindungen der Formel (I), welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure, Salze.

Eine Ausführungsform des erfindungsgemäßen Verfahrens ist dadurch gekennzeichnet, daß man Aldehyde der Formel VII durch Behandlung mit dem Niob (III)-Komplex NbCl₃ (DME) in inerten Lösungsmitteln im Temperaturbereich von -78 °C bis zur Siedetemperatur des Reaktionsgemisches stereoselektiv reduktiv zu den Verbindungen der Formel I dimerisiert.

Der obengenannte Niobkomplex wird aus der Reaktion von NbCl₅ mit Bu₃ SnH isoliert (J. Am. Chem. Soc. 109, 6551 (1987)); weiterhin ist er kommerziell erhältlich (z. B. Aldrich Chemie GmbH, Steinheim).

Eine bevorzugte Ausführungsform zur Herstellung der Verbindungen der Formel I mit den obengenannten bevorzugten Konfigurationen ist dadurch gekennzeichnet, daß man NbCl₃ (DME) in einer schutzgasgespülten Apparatur (z.B. N₂ oder Argon) in inerten Lösungsmitteln, wie cyclischen oder acyclischen Dialkylethern, aromatischen- oder Alkylkohlenwasserstoffen, oder halogenierten Kohlenwasserstoffen, insbesondere Dichlormethan, Di-, Tri- oder Tetrachlorethan, Toluol, THF bei Temperaturen von -78 °C bis Siedetemperatur des Lösungsmittels, bevorzugt von 0 °C bis zur Siedetemperatur des Lösungsmittels vorlegt und bezogen auf den Niob (III)-Komplex mit 0,3 bis 1,0, vorzugsweise 0,7 bis 0,9 Äquivalenten Aldehyd der allgemeinen Formel VII versetzt und bis zur Reaktionsvervollständigung nach DC-Kontrolle unter Schutzgasatmosphäre (z.B. N₂ oder Argon) bei der jeweiligen Anfangstemperatur rührt. Die Umsetzung erfolgt in besonderem Maße stereoselektiv bei Benutzung des Lösungsmittels THF.

Zur Aufarbeitung wird die Reaktionstemperatur vorzugsweise auf Zimmertemperatur gestellt und die Mischung mit der wäßrigen Lösung eines Komplexbildners, vorzugsweise 10 - 30 %iger wäßriger Zitronensäure- oder Tartratlösung versetzt. Nach der Phasentrennung wird die wäßrige Phase mit im Reaktionsansatz verwendetem Lösungsmittel oder ersatzweise mit einem mit Wasser nicht mischbaren, organischen Lösungsmittel extrahiert, die vereinigten organischen Phasen sukzessive mit wäßriger Base, wäßriger Säure und Wasser gewaschen, getrocknet, filtriert und zur Trockne eingedampft, wonach das Rohprodukt in Ausbeuten von 20% bis 90% d.Th. anfällt. Die Reinigung erfolgt bevorzugt durch Kristallisation oder Chromatographie an einer Kieselgelsäule oder entfällt aufgrund der ausreichenden Reinheit des anfallenden Rohproduktes.

Die besonders bevorzugten Verbindungen der Formel I in der SRRS-Konfiguration erhält man in besonders hoher Ausbeute aus den α-Aminoaldehyden der Formel VII mit S-Konfiguration durch erfindungsgemäße Reaktion bei Raumtemperatur bis Siedetemperatur des Reaktionsgemisches, insbesondere bei Siedetemperatur des Reaktionsgemisches.

Optisch reine α-Aminoaldehyde der Formel VII werden in einfacher, literaturbekannter Weise aus Aminosäuren erhatten, z.B. wie nachfolgend näher erläutert.

Kommerziell erhältliche oder selbst synthetisierte Verbindungen der Formel IX 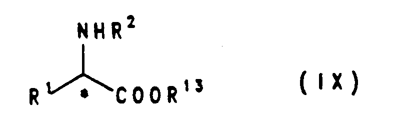
worin
- R¹³: H, (C₁-C₄)-Alkyl, (C₆-C₁₄)-Aryl oder (C₆-C₁₄)-Aryl-(C₁-C₆)-alkyl, insbesondere Methyl, Ethyl oder Benzyl bedeutet und
- R¹ und R²: wie oben definiert sind
(Houben Weyl 15/1 und 2, Stuttgart, 1974; V. Teetz, R. Geiger, H. Gaul, Tetrahedron Letters 25(40), 4479 (1984), A. Pictet, T. Spengler, Chem. Ber. 44, 2030 (1911); R.M. Williams, "Synthesis of Optically Active α-Aminoacids", Pergamon Press, Oxford, 1989) werden analog zu literaturbekannten Methoden (M.W. Drewes, "The Syntheses and Stereoselektive Reactions of α-Aminoaldehydes", Inauguraldissertation, Fachbereich Chemie der Philipps-Universität, Marburg/Lahn 1988; und darin zitierte Lit.; N.G. Gaylord,"Reduktion with Complex Metal Hydrides", Interscience Publishers, NY London, 1956; H. Schenker, Angew. Chemie 73, 81 (1961); C.F. Stanfield, J.E. Parker, P. Kanellis, J. Org. Chem.46, 4797 und 4799 (1981); K.E. Rittle, C.F. Homnick, B.E. Evans, J. Org. Chem.47, 3016 (1982); K. Haaf, C. Rüchardt, Chem. Ber.123, 635(1990)) beispielsweise mit NaBH₄ (N.G. Gaylord, s.o.), BH₃·THF (K.E. Rittle, s.o.), oder LiAlH₄ (K. Haaf, s.o.) in inerten Lösungsmitteln, oder niederen Alkoholen, oder alkoholisch-wäßrigen Mischungen zu den Aminoalkoholen der Formel X reduziert, 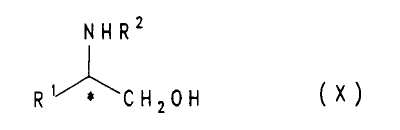
wobei
R¹ und R² wie oben definiert sind. Anschließend werden die so erhaltenen Verbindungen der Formel X nach bekannten Verfahren (Houben Weyl, s.o.; E. Gross, J. Meienhofer, Ed., "The Peptides - Protection of Functional Groups in Peptide Synthesis", Academic Press, New York, 1981; T.W. Greene, "Protective Groups in Organic Synthesis", John Wiley & Sons, NY Chichester Brisbane Toronto Singapore, 1980; Proceedings of European Peptide Symposium, Platja D'Aro, September 1990 zu den Verbindungen der Formel XI 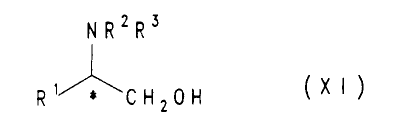
mit den genannten Bedeutungen für R¹ - R³ umsetzt und anschließend mit Pyridiniumdichromat (C.F. Stanfield, s.o.), CrO₃·Pyridin (K.E. Rittle, s.o.), insbesondere aber mit (COCl)₂ und DMSO und nach der Methode von Swern racemisierungsfrei zu den Aldehyden der Formel VII 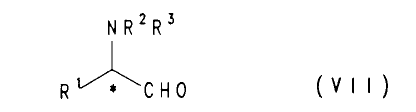
wobei R¹, R² und R³ wie oben definiert sind, oxidiert (K. Omura, A.K. Sharma, D. Swern, J. Org. Chem. 41, 957 (1976); D. Swern, S.L. Huang, A.J. Mancuso, J. Org. Chem. 43, 2480 (1978); A.J. Mancuso, D. Swern, Synthesis, 165 (1981)).

Eine zweite Variante besteht darin daß man Verbindungen der Formel IX analog zu den oben genannten literaturbekannten Synthesen zu Verbindungen der Formel XII 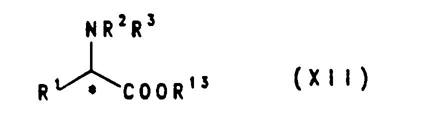
worin R¹, R², R³ und R¹³ wie oben definiert sind, umsetzt und diese - nach eventuell vorangehender racemisierungsfreier Verseifung eines Esters der Formel XII mit R¹³ ≠ H - beispielsweise nach der Methode von Weinreb (S. Nahm, S.M. Weinreb, Tetrahedron Letters 22, 3815 (1981)) durch Umsetzung mit N,O-Dimethylhydroxylamin zu den Verbindungen der Formel XIII umsetzt, 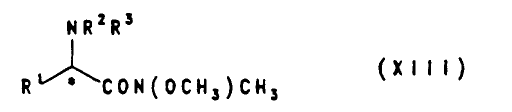
wobei R¹, R², R³ wie oben definiert sind. Die Amide der allgemeinen Formel XIII werden zum Beispiel nach der Methode von Castro (J.A. Fehrentz, B. Castro, Synthesis, 676 (1983); J.A. Fehrentz, B. Castro, Int. J. Peptide Protein Res. 26, 236 (1985)) durch die Reduktion mit LiAlH₄ direkt und racemisierungsfrei in die genannten Aldehyde der allgemeinen Formel VII überführt.

In einer dritten Variante werden Carbonsäuren der allgemeinen Formel XII (R¹³ = H) mit Thionylchlorid oder anderen geeigneten Halogenierungsmitteln in die entsprechenden Carbonsäurehalogenide der Formel XIV 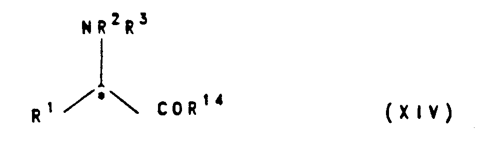
wobei
- R¹ - R³: den obigen Definitionen entspricht und
- R¹⁴: Cl, Br, I oder Reste gemischter Carbonsäureanhydride bedeutet,
derivatisiert und nachfolgend mit H₂/Pd/BaSO₄ racemisierungsfrei zu den Aldehyden der allgemeinen Formel VII reduziert (analog: R.L. Johnson, J. Med. Chem. 25, 605 (1982)). Im Prinzip können Aldehyde aus Carbonsäuren und deren Derivaten auch mit anderen Methoden hergestellt werden, etwa durch Umsetzung mit einfachen und komplexen Metallhydriden, Metallcarbonylkomplexen, Silanen, Alkalimetallen, Formiaten oder photochemisch (Houben Weyl 7E3, 418ff, Stuttgart, 1983).

Im Gegensatz zu den bei Pedersen et al. beschriebenen Kupplungen mit Niobkomplexen (J. Am. Chem. Soc. 109, 3152 (1987) und 109, 6551 (1987) werden im vorliegenden Verfahren Reaktionen ohne Iminoaldehydkomponente durchgeführt, des weiteren wird bei dem vorliegenden Verfahren eine gleichzeitige Kontrolle über vier Stereozentren ausgeübt. Beim Einsatz von optisch aktiven Ausgangsmaterialien werden dabei optisch aktive Kopplungsprodukte in hoher Ausbeute gewonnen. Ein weiterer Vorteil des hier beschriebenen Verfahrens ist die größere Selektivität des Reduktionsmittels für die aktivierte Aldehydfunktion, die zu größerer Kompatibilität mit anderen funktionellen Gruppen führt. Wie J.E. McMurry (Chem. Rev. 89, 1513 (1989), insbesondere Tabelle 2, S. 1515) beschreibt, ist das McMurry-Reagenz nur teilweise kompatibel mit funktionellen Gruppen wie zum Beispiel Amid, Carbonsäure, Ester, Keton und inkompatibel mit funktionellen Gruppen wie Nitro, Oxim, Sulfoxid, Epoxid und 1,2-Diol. Hingegen ist der Niobkomplex zum Beispiel vollständig kompatibel mit der Amidfunktion und auch andere nicht aktivierte Carboxylfunktionen reagieren nicht mit nennenswerter Geschwindigkeit (J. Am. Chem. Soc. 109, 6551 (1987)).

### Verwendete Abkürzungen:

- Cha: Cyclohexylalanin
- Chg: Cyclohexylglycin
- DABCO: 1,4-Diazabicyclo[2.2.2]octan
- DME: Dimethoxyethan
- DMF: Dimethylformamid
- DMSO: Dimethylsulfoxid
- EDTA: Ethylendiamintetraessigsäure
- HMPA: Hexamethylphosphorsäuretriamid
- MTB: Methyl-tert. butyl
- Nal: 1- und 2-Naphthylalanin
- Npg: Neopentylglycin
- Tbg: Tert. Butylglycin
- THF: Tetrahydrofuran
- Thia: 2-Thienylalanin
- TMEDA: N,N,N',N'-Tetramethylethylendiamin

Durch die folgenden Beispiele wird das erfindungsgemäße Verfahren weiter illustriert und es werden konkrete Durchführungsvarianten geschildert. Diese Beispiele und Durchführungsvarianten limitieren den Erfindungsgegenstand weder bezüglich der strukturellen Bandbreite der auf diese Weise diastereoselektiv reduktiv dimerisierten α-Aminoaldehyde VII, noch bezüglich der Verfahrensbedingungen (Reagenzherstellung, physikalische Parameter der reduktiven Dimerisierung, Lösungsmittel, Reaktionszeit, Aufarbeitung, Reinigung und Analytik der Reaktionsprodukte).

### Beispiel 1:

### N-(tert.-butoxycarbonylamino)-(S)-Phenylalanin-N-methoxy-N-methylamid

88,1 g (332 mmol) (S)-Phenylalanin werden in 1,2 l Dichlormethan gelöst und unter Stickstoffatmosphäre bei einer konstanten Innentemperatur von 20 °C (Kühlung durch Eisbad) mit 268 ml (2,1 mol) Ethylmorpholin und 43,7 g (445 mmol) N,O-Dimethylhydroxylamin Hydrochlorid versetzt. Das Reaktionsgemisch wird auf -10 °C abgekühlt und eine Lösung von 252 ml Propanphosphonsäureanhydrid in 250 ml Essigsäureethylester zugetropft. Durch Nachrühren (1 h bei 0 °C, anschließend 2 h bei Raumtemperatur) wird vollständiger Umsatz der Reaktanden erreicht. Man wäscht mit 1 l 3 N HCl, 800 ml gesättigter, wäßriger NaHCO₃-Lösung, 800 ml gesättigter wässriger NaCl-Lösung und trocknet die organische Phase über Na₂SO₄. Nach dem Verdampfen des Lösungsmittels verbleiben 100 g eines farblosen Öles, daß ohne weitere Reinigung der Reduktion zum entsprechenden Aminoaldehyd zugeführt werden kann.

### Beispiel 2 :

### N-(tert.-Butoxycarbonyl)-(S)-Phenylalaninal

4,36 g Lithiumaluminiumhydrid werden in 875 ml trockenem Diethylether unter Stickstoffatmosphäre bei 0 °C vorgelegt und unter Rühren eine Lösung von 26,9 g N-(tert.-Butoxycarbonylamino)-(S)-Phenylalanin-N-methoxy-N-methylamid in 73 ml Diethylether zugegeben. Es wird 30 min bei 0 °C gerührt und danach mit 450 ml 5%iger kalter wäßriger KHSO₄-Lösung versetzt. Die Phasen werden getrennt, die organische Phase nacheinander mit 300 ml 0,5 N HCl, 600 ml gesättigter wäßriger NaHCO₃-Lösung, 600 ml gesättigter wäßriger NaCl-Lösung gewaschen und schließlich über Na₂SO₄ getrocknet. Nach dem Verdampfen des Lösungsmittels verbleiben 20,9 g (96,3 %) weiße Kristalle, die ohne weitere Aufreinigung zur reduktiven Kupplung eingesetzt werden können.

### Beispiel 3 :

### (N-(tert.-Butoxycarbonylvalinyl)amino)-(S)-Phenylalaninal

2,1 ml (25 mmol) Oxalylchlorid werden unter Inertgasatmosphäre in 125 ml trockenem Dichlormethan gelöst. Bei -70 °C tropft man unter Rühren 2,4 ml (33,4 mmol) DMSO ein und fügt nach 15 min Wartezeit eine Lösung von 5,85 g (16,7 mmol) N-(tert.-Butoxycarbonyl)-(S)-Valyl-(S)-Phenylalaninol in einer Mischung aus 4 ml DMSO und 30 ml Dichlormethan langsam zu. Das Reaktionsgemisch wird 30 min bei -70 °C nachgerührt und dann 9,4 ml (66,8 mmol) Triethylamin eingetropft, wobei die Temperatur auf -60 °C ansteigt. Nach weiteren 15 min bei -60 °C hydrolysiert man mit 200 ml 15% iger wäßriger Zitronensäure und trennt die Phasen. Die organische Phase wird nacheinander mit jeweils 200 ml gesättigter wäßriger Bicarbonatlösung, mit Wasser, schließlich mit gesättigter wäßriger Kochsalzlösung gewaschen und über Natriumsulfat getrocknet. Nach dem Verdampfen des Lösungsmittels verbleiben 4,4 g weiße Kristalle die ohne weitere Reinigungschritte der Dimerisierung zugeführt werden.

Allgemeine Versuchsvorschrift der reduktiven Kupplung:
1,4 eq. Trichlor-(Dimethoxyethan)-Niob (III) werden unter Inertgasatmosphäre in 10 ml trockenem Lösungsmittel vorgelegt, mit 1 eq. Aldehyd in 2 ml trockenem LM versetzt und die Reaktionstemperatur eingestellt. Bei stabiler Temperatur wird der Reaktionsumsatz mittels DC-Kontrolle überwacht. Nach Ablauf der Reaktion liefert Ausschütteln bei Raumtemperatur mit wäßriger Natriumtartratlösung und wäßriger Zitronensäurelösung, sukzessives Waschen der organischen Phase mit wäßriger Basenlösung, wäßrige Säure und Wasser, Trocknen der organischen Phase über Na₂SO₄, Abfiltrieren vom Trockenmittel und Entfernen des Lösungsmittels einen öligen oder festen Rückstand, der gegebenenfalls durch Chromatographie an Kieselgel und/oder durch Kristallisation gereinigt wird.

### Beispiel 4 :

### N,N'-Bis-(tert.-Butoxycarbonyl)-2S,5S-diamino-1,6-diphenyl-hexan-3R,4R-diol

Ausgehend von 2,5 g N-(tert.-Butoxycarbonyl)-(S)-phenylalaninal erhält man 1,5 g Kristalle der Titelverbindung analog der allgemeinen Vorschrift (Lösungsmittel: THF; Reaktionstemperatur: Rückfluß).
- MS (FAB):: 507 (M+Li⁺), 401, 352, 307

### Beispiel 5 :

### (2S, 3R, 4R, 5S)-2,5-(N,N'-((tert.-Butoxycarbonyl)-(S)valinyl)amino)-3,4-dihydroxy-1,6-diphenylhexan

Ausgehend von 3,5 g N-(tert.-Butoxycarbonyl)-(S)-valinyl-phenylalaninal erhält man analog der allgemeinen Vorschrift 1,9 g Öl der Titelverbindung.
(Lösungsmittel: THF; Reaktionstemperatur: Rückfluß).
- MS (FAB):: 705 (M+Li⁺), 699 (M+H⁺), 667, 605, 599.

### Beispiel 6 :

### 1,2-Bis[N-{2(S)-(1,1-dimethylethylsulfonylmethyl)-3-(1-naphthyl)-propionyl}-(S)-valyl]-(S)-1,2,3,4-tetrahydroisochinolin-3-yl]-ethan-1(R),2(R)-diol

Ausgehend von 2,5 g N[{(S)-2-(1,1-Dimethylethylsulfonylmethyl)-3-(1-naphthyl)propionyl}-(S)-valyl]-(S)-1,2,3,4-tetrahydroisochinolin-3-carbaldehyd erhält man 0,8 g gelbliche Kristalle der Titelverbindung analog der Versuchsvorschrift unter in situ - Darstellung des Kupplungsreagenzes (Komplexbildner: 1,3-Dimethylimidazolidin-2-on).
- MS (FAB):: 1162 (M+Li⁺), 1156 (M+H⁺), 741, 388.

### Beispiel 7:

### N,N'-Bis[{(S)-2-(1,1-dimethylethylsulfonylmethyl)-3-(1-naphthyl)-propionyl}-(S)-valyl]-2(S),5(S)-diamino-1,6-diphenylhexan-3(R),4(R)-diol

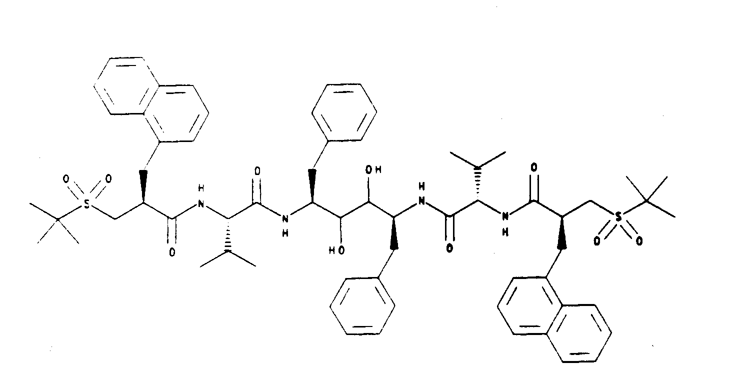

Ausgehend von 2,0 g N[{(S)-2-(1,1-Dimethylethylsulfonylmethyl)-3-(1-naphthyl)propionyl}-(S)-valyl]-(S)-phenylalaninal erhält man 0,8 g Kristalle der Titelverbindung analog der allgemeinen Vorschrift.
(Lösungsmittel: THF; Reaktionstemperatur: Rückfluß).
- MS (FAB):: 1154 (M+Na⁺), 1132 (M+H⁺), 716, 567.

## Patentansprüche

1. Verfahren zur Herstellung von Verbindungen der Formel I 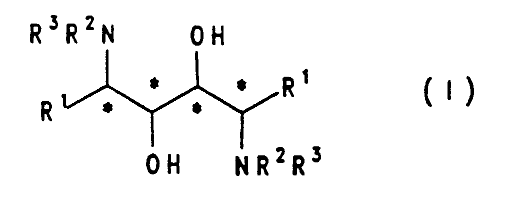 worin
R¹ für einen Seitenkettenrest einer natürlichen oder nicht-natürlichen α-Aminosäure steht;
R² und R³ gleich oder verschieden sind und
a)
- Wasserstoff
b)
- einen Rest der Formel
D-(E)ₙ-(F)ₒ-(G)ₚ (II)
bedeuten, wobei E, F und G unabhängig voneinander für eine natürliche oder unnatürliche Aminosäure, Azaaminosäure oder Iminosäure stehen;
n, o, p unabhängig voneinander 0 oder 1 bedeuten;
D für R⁴ oder einen Rest der Formeln III, IV oder V steht 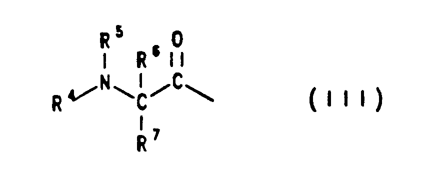 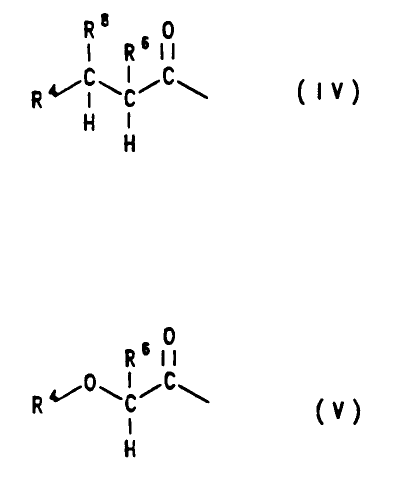 worin R⁴ bedeutet
b₁)
- Wasserstoff,
- Carboxyl,
- (C₁-C₁₈)-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- (C₁-C₇)-Alkoxy,
- Carbamoyl,
- (C₁-C₈)-Alkanoyloxy,
- Carboxy,
- (C₁-C₇)-Alkoxycarbonyl,
- F, Cl, Br, I,
- Amino,
- Amidino, das gegebenenfalls durch einen, zwei oder drei (C₁-C₈)-Alkylreste substituiert sein kann,
- Guanidino, das gegebenenfalls durch einen oder zwei Benzyloxycarbonylreste oder durch einen, zwei, drei oder vier (C₁-C₈)-Alkylreste substituiert sein kann,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio,
- Hydroxamino,
- Hydroximino,
- Sulfamoyl,
- Sulfo,
- Carboxamido,
- Formyl,
- Hydrazono,
- Imino,
- einen Rest CONR⁹R¹⁰,
- durch bis zu sechs Hydroxy oder
- durch bis zu fünf (C₁-C₈)-Alkanoyloxy substituiert ist;
- mono-, bi- oder tricyclisches (C₃-C₁₈)-Cycloalkyl,
- (C₃-C₁₈)-Cycloalkyl-(C₁-C₆)-alkyl,
wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- (C₁-C₇)-Alkoxy,
- (C₁-C₇)-Alkyl,
- (C₁-C₇)-Alkyloxycarbonyl,
- Amino,
- (C₁-C₆)-Alkylamino-(C₁-C₆)-alkyl,
- Di-(C₁-C₆)-Alkylamino-(C₁-C₆)-alkyl,
- Amidino,
- Hydroxamino,
- Hydroximino,
- Hydrazono,
- Imino,
- Guanidino,
- (C₁-C₆)-Alkoxysulfonyl,
- (C₁-C₆)-Alkoxysulfinyl,
- (C₁-C₆)-Alkoxycarbonylamino,
- (C₆-C₁₂)-Aryl-(C₁-C₄)-alkoxycarbonylamino,
- (C₁-C₇)-Alkylamino,
- Di-(C₁-C₇)-alkylamino und
- Trifluormethyl substituiert ist;
- (C₆-C₁₄)-Aryl,
- (C₆-C₁₄)-Aryl-(C₁-C₆)-alkyl,
- (C₆-C₁₄)-Aryloxy-(C₁-C₆)-alkyl oder
- (C₆-C₁₄)-Aryl-(C₃-C₈)-cycloalkyl,
worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-(C₁-C₄)-alkyl,
- Trifluormethyl,
- Formyl,
- Carboxamido,
- Mono- oder Di- (C₁-C₄)-alkylaminocarbonyl,
- Nitro,
- (C₁-C₇)-Alkoxy,
- (C₁-C₇)-Alkyl,
- (C₁-C₇)-Alkoxycarbonyl,
- Amino,
- (C₁-C₇)-Alkylamino,
- Di-(C₁-C₇)-alkylamino,
- Carboxy,
- Carboxymethoxy,
- Amino-(C₁-C₇)-alkyl,
- (C₁-C₇)-Alkylamino-(C₁-C₇)-alkyl,
- Di-(C₁-C₇)-alkylamino-(C₁-C₇)-alkyl,
- (C₁-C₇)-Alkoxycarbonylmethoxy,
- Carbamoyl,
- Sulfamoyl,
- (C₁-C₇)-Alkoxysulfonyl,
- (C₁-C₈)-Alkylsulfonyl,
- Sulfo-(C₁-C₈)-alkyl,
- Guanidino-(C₁-C₈)-alkyl und
- (C₁-C₆)-Alkoxycarbonylamino substituiert ist;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl,
wobei Het jeweils für den Rest eines 5-bis 7-gliedrigen monocyclischen oder 8- bis 10-gliedrigen bicyclischen Ringsystems steht, das benzanelliert, aromatisch, teil-oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter b₁) definiert und/oder mit Oxo mono-, di- oder trisubstituiert ist, oder einen Rest NR⁹R¹⁰ bedeutet, oder
b₂)
- einen Rest der Formel VI
R^{4a} - W (VI)
worin R^{4a} wie R⁴ unter b₁) definiert ist und W für -CO-, -CS-, O-CO-, -SO₂-, -SO-, -S-, -NHSO₂-, -NHCO-, -CH(OH)-, -N(OH)-oder -CO-V-, wobei V ein Peptid mit insgesamt 1 bis 10 Amino-, Imino-und/oder Azaaminosäuren bedeutet, steht;
oder worin R⁴ zusammen mit R⁸ und den diese tragenden Atomen mono-oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
b₃)
- einen Glycosylrest, bevorzugt einen Glucofuranosyl-oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet; oder
b₄)
- eine Aminoschutzgruppe;
R⁵
- Wasserstoff oder
- (C₁-C₈)-Alkyl bedeutet, oder
- zusammen mit R⁶ und den diesen Rest tragenden Atomen mono-oder bicyclische, gesättigte oder teilweise ungesättigte Ringsysteme mit 5-12 Ringgliedern bildet;
R⁶
- wie R⁴ unter b₁) definiert ist;
- für Hydroxy oder (C₁-C₄)-Alkanoyloxy steht; oder
- zusammen mit R⁷ und den diesen Rest tragenden Atomen cyclische, gesättigte oder teilweise ungesättigte Ringsysteme mit 3 bis 12 Ringgliedern bildet; oder
- zusammen mit R⁸ und den diese tragenden Atomen ein mono-oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
R⁷
- Wasserstoff oder
- (C₁-C₆)-Alkyl bedeutet;
R⁸
- Wasserstoff,
- Hydroxy,
- (C₁-C₄)-Alkanoyloxy oder
- (C₁-C₈)-Alkyl bedeutet;
R⁹ und R¹⁰
- Wasserstoff,
- (C₁-C₈)-Alkyl, das durch
- Amino,
- (C₁-C₄)-Alkylamino,
- Di-(C₁-C₄)-alkylamino,
- Mercapto,
- Carboxy,
- Hydroxy oder
- (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-alkoxycarbonyl, die im Arylteil wie bei R⁴ beschrieben, substituiert sein können,
- Het oder
- Het-(C₁-C₄)-alkyl, wobei Het wie bei R⁴ beschrieben definiert ist, bedeuten oder wobei
R⁹ und R¹⁰ zusammen mit dem sie tragenden Stickstoffatom monocyclische oder bicyclische, gesättigte, teilweise ungesättigte oder aromatische Ringsysteme bilden, die als Ringglieder neben Kohlenstoff noch 1 oder weitere 2 Stickstoffatome, 1 Schwefelatom oder 1 Sauerstoffatom enthalten und durch (C₁-C₄)-Alkyl substituiert sein können, wobei in den vorstehenden Verbindungen der Formel I eine oder mehrere Amidgruppen (-CONH-) der Hauptkette ersetzt sein können durch -CH₂NR¹¹-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹²)CH₂-und -P(O)(OR¹²)NH-, oder auch durch eine Amidgruppe mit umgekehrter Polarität (-NHCO-);
worin R¹¹ und R¹² unabhängig voneinander stehen für
- Wasserstoff oder
- (C₁-C₄)-Alkyl;
sowie deren Enantiomere und physiologisch verträgliche Salze, dadurch gekennzeichnet, daß homochirale α-Aminoaldehyde der Formel VII 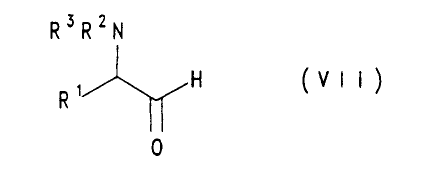 worin R¹, R² und R³ wie oben definiert sind,
mit dem NbCl₃-Dimethoxyethan-Komplex [NbCl₃ (DME)] behandelt werden, wobei eine gleichzeitige Kontrolle über alle vier Chiralitätszentren vorliegt.

2. Verfahren zur Herstellung von Verbindungen der Formel I gemäß Anspruch 1, dadurch gekennzeichnet, daß
R¹ einen Seitenkettenrest der α-Aminosäuren Gly, Ala, Val, Leu, Ile, Ser, Thr, Cys, Met, Pro, Lys, Arg, His, Asp, Asn, Glu, Gln, Phe, Tyr, Trp oder Cha bedeutet;
R² und R³ gleich oder verschieden sind und
a)
- Wasserstoff
b)
- einen Rest der Formel II
worin o und p = 0 ,
n = 0 oder 1 ist und
E für eine der oben genannten α-Aminosäuren steht,
D für R⁴ oder einen Rest der Formeln III oder IV steht, worin R⁴
b₁)
- Wasserstoff
- (C₁-C₉)-Alkyl, das gegebenenfalls einfach oder zweifach ungesättigt ist und das gegebenenfalls durch bis zu 3 gleiche oder verschiedene Reste aus der Reihe
- Hydroxy,
- (C₁-C₇)-Alkoxy,
- Carbamoyl,
- (C₁-C₈)-Alkanoyloxy,
- (C₁-C₇)-Alkoxycarbonyl,
- F, Cl,
- Amino,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio subsitutiert ist,
- (C₆-C₁₄)-Aryl,
- (C₆-C₁₄)-Aryl-(C₁-C₆)-alkyl oder
- (C₆-C₁₄)-Aryloxy-(C₁-C₆)-alkyl, worin der Arylteil jeweils gegebenenfalls durch einen, zwei oder drei gleiche oder verschiedene Reste aus der Reihe der oben genannten bevorzugten Substituenten von (C₁-C₉)-Alkyl substituiert sein kann,
b₂)
- einen Rest der Formel VI, worin
R^{4a} wie R⁴ unter b₁) definiert ist und W für -CO-, O-CO-, -SO₂-, -SO-, -S-, -NHCO-, -CH(OH)- steht;
b₄)
- eine Aminoschutzgruppe Fmoc, Z oder Boc bedeutet,
R⁵ und R⁷ Wasserstoff bedeuten,
R⁶
- wie R⁴ definiert ist, und
R⁸
- Wasserstoff,
- Hydroxy,
- (C₁-C₄)-Alkanoyloxy oder
- (C₁-C₈)-Alkyl bedeutet.

3. Verfahren zur Herstellung von Verbindungen der Formel I gemäß einem der Ansprüche 1 oder 2, dadurch gekennzeichnet, daß einer der Reste R² oder R³ Wasserstoff bedeutet.

4. Verfahren zur Herstellung von Verbindungen der Formel I gemäß einem der Ansprüche 1 bis 3, dadurch gekennzeichnet, daß die Verbindungen der Formel I SRRS-Konfiguration oder RSSR-Konfiguration haben.

5. Verfahren zur Herstellung von Verbindungen der Formel I gemäß einem der Ansprüche 1 bis 4, dadurch gekennzeichnet, daß
R¹
- einen Seitenkettenrest der α-Aminosäuren Ala, Val, Leu, Ile, Pro, Phe, Cha oder Tyr bedeutet,
R² und R³ gleich oder verschieden sind und
a)
- Wasserstoff
b)
- einen Rest der Formel II, worin
o und p = 0 sind,
n 0 oder 1 bedeutet und
E für Ala, Val, Leu, Ile, Pro, Phe, Cha oder Tyr steht;
D für R⁴ oder einen Rest der Formel IV steht mit R⁴
b₁)
- Wasserstoff,
- (C₁-C₄)-Alkyl,
- Phenyl oder Naphthyl,
- Phenylmethyl oder Naphthylmethyl,
b₂)
- Rest der Formel VI, worin R^{4a} wie R⁴ unter b₁) definiert ist und W für -CO-, -O-CO-, -SO₂-, -SO-, -S-, -NHCO-, -CH(OH)- steht, oder
b₄)
- eine Aminoschutzgruppe Fmoc, Z oder Boc bedeuten,
R⁵, R⁷ und R⁸ Wasserstoff bedeuten, und
R⁶ wie R⁴ unter b₁) definiert ist.

6. Verfahren zur Herstellung von Verbindungen der Formel I gemäß einem der Ansprüche 1 bis 5, dadurch gekennzeichnet, daß zur Herstellung einer Verbindung der Formel I in der SRRS-Konfiguration eine Verbindung der Formel VII mit S-Konfiguration bei einer Temperatur zwischen Raumtemperatur und Siedetemperatur des Reaktionsgemisches zur Reaktion gebracht wird.

7. Verfahren zur Herstellung von Verbindungen der Formel I gemäß einem der Ansprüche 1 bis 6, dadurch gekennzeichnet, daß eine Verbindung der Formel VII in Gegenwart von THF als Lösungsmittel mit dem NbCl₃-Dimethoxyethan-Komplex behandelt wird.

## Claims

1. A process for the preparation of compounds of the formula I 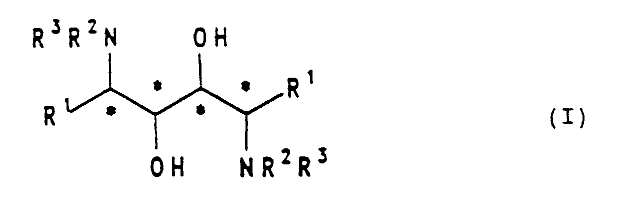 in which
R¹ is a side-chain radical of a natural or unnatural α-amino acid;
R² and R³ are identical or different and are
a)
- hydrogen
b)
- a radical of the formula
D-(E)ₙ-(F)ₒ-(G)ₚ (II)
where E, F and G independently of one another are a natural or unnatural amino acid, aza amino acid or imino acid;
n, o and p independently of one another are 0 or 1;
D is R⁴ or a radical of the formula III, IV or V 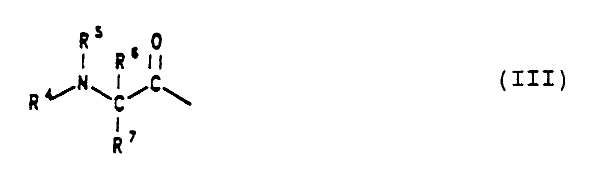 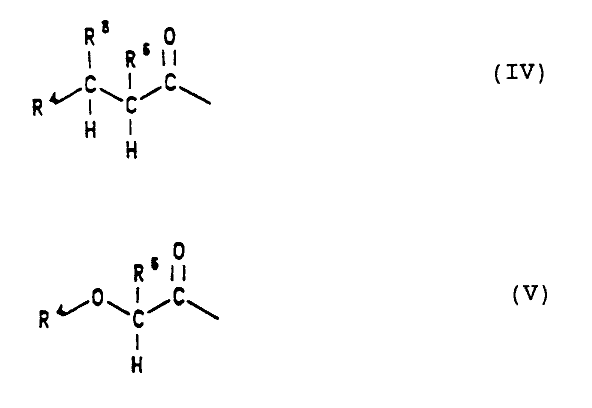 in which R⁴ is
b₁)
- hydrogen,
- carboxyl,
- (C₁-C₁₈)-alkyl which is optionally monounsaturated or di-unsaturated and which is optionally substituted by up to 3 identical or different radicals selected from the group comprising
- mercapto,
- hydroxyl,
- (C₁-C₇)-alkoxy,
- carbamoyl,
- (C₁-C₈)-alkanoyloxy,
- carboxyl,
- (C₁-C₇)-alkoxycarbonyl,
- F, Cl, Br or I,
- amino,
- amidino which can optionally be substituted by one, two or three (C₁-C₈)-alkyl radicals,
- guanidino which can optionally be substituted by one or two benzyloxycarbonyl radicals or by one, two, three or four (C₁-C₈)-alkyl radicals,
- (C₁-C₇)-alkylamino,
- di-(C₁-C₇)-alkylamino,
- (C₁-C₆)-alkoxycarbonylamino,
- (C₇-C₁₅)-aralkoxycarbonyl,
- (C₇-C₁₅)-aralkoxycarbonylamino,
- phenyl-(C₁-C₄)-alkoxy,
- 9-fluorenylmethoxycarbonylamino,
- (C₁-C₆)-alkylsulfonyl,
- (C₁-C₆)-alkylsulfinyl,
- (C₁-C₆)-alkylthio,
- hydroxamino,
- hydroximino,
- sulfamoyl,
- sulfo,
- carboxamido,
- formyl,
- hydrazono,
- imino,
- a radical CONR⁹R¹⁰,
- by up to six hydroxyl or
- by up to five (C₁-C₈)-alkanoyloxy;
- mono-, bi- or tricyclic (C₃-C₁₈)-cycloalkyl,
- (C₃-C₁₈)-cycloalkyl-(C₁-C₆)-alkyl,
where the cycloalkyl moiety is in each case optionally substituted by one or two identical or different radicals selected from the group comprising
- F, Cl, Br or I,
- carboxyl,
- carbamoyl,
- carboxymethoxy,
- hydroxyl,
- (C₁-C₇)-alkoxy,
- (C₁-C₇)-alkyl,
- (C₁-C₇)-alkoxycarbonyl,
- amino,
- (C₁-C₆)-alkylamino-(C₁-C₆)-alkyl,
- di-(C₁-C₆)-alkylamino-(C₁-C₆)-alkyl,
- amidino,
- hydroxamino,
- hydroximino,
- hydrazono,
- imino,
- guanidino,
- (C₁-C₆)-alkoxysulfonyl,
- (C₁-C₆)-alkoxysulfinyl,
- (C₁-C₆)-alkoxycarbonylamino,
- (C₆-C₁₂)-aryl-(C₁-C₄)-alkoxycarbonylamino,
- (C₁-C₇)-alkylamino,
- di-(C₁-C₇)-alkylamino and
- trifluoromethyl;
- (C₆-C₁₄)-aryl,
- (C₆-C₁₄)-aryl-(C₁-C₆)-alkyl,
- (C₆-C₁₄)-aryloxy-(C₁-C₆)-alkyl or
- (C₆-C₁₄)-aryl-(C₃-C₈)-cycloalkyl,
in which the aryl moiety is in each case optionally substituted by one, two or three identical or different radicals selected from the group comprising
- F, Cl, Br or I,
- hydroxyl,
- mono, di- or trihydroxy-(C₁-C₄)-alkyl,
- trifluoromethyl,
- formyl,
- carboxamido,
- mono- or di- (C₁-C₄)-alkylaminocarbonyl,
- nitro,
- (C₁-C₇)-alkoxy,
- (C₁-C₇)-alkyl,
- (C₁-C₇)-alkoxycarbonyl,
- amino,
- (C₁-C₇)-akylamino,
- di-(C₁-C₇)-alkylamino,
- carboxyl,
- carboxymethoxy,
- amino-(C₁-C₇)-alkyl,
- (C₁-C₇)-alkylamino-(C₁-C₇)-alkyl,
- di-(C₁-C₇)-alkylamino-(C₁-C₇)-alkyl,
- (C₁-C₇)-alkoxycarbonylmethoxy,
- carbamoyl,
- sulfamoyl,
- (C₁-C₇)-alkoxysulfonyl,
- (C₁-C₈)-alkylsulfonyl,
- sulfo-(C₁-C₈)-alkyl,
- guanidino-(C₁-C₈)-alkyl and
- (C₁-C₆)-alkoxycarbonylamino;
- het,
- het-(C₁-C₆)-alkyl,
- het-(C₃-C₈)-cycloalkyl,
- et-(C₃-C₈)-cycloalkyl-(C₁-C₄)-alkyl,
- het-(C₃-C₈)-cycloalkoxy-(C₁-C₄)-alkyl,
- het-thio-(C₁-C₆)-alkyl,
- het-thio-(C₃-C₈)-cycloalkyl,
- het-thio-(C₃-C₈)-cycloalkyl-(C₁-C₄)-alkyl,
where het in each case is the radical of a 5- to 7-membered monocyclic or 8- to 10-membered bicyclic ring system which can be benzo-fused, aromatic, partly or completely hydrogenated, which as hetero elements can contain one, two, three or four different radicals from the group comprising N, O, S, NO, SO and SO₂, which can be substituted by 1 to 6 hydroxyl and which is optionally defined as in the case of (C₆-C₁₄)-aryl in b₁) and/or is mono-, di- or trisubstituted by oxo, or is a radical NR⁹R¹⁰, or
b₂)
- a radical of the formula VI
R^{4a} - W (VI)
in which R^{4a} is defined as R⁴ in b₁) and W is - CO-, -CS-, -O-CO-, -SO₂-, -SO-, -S-, -NHSO₂-, -NHCO-, -CH(OH)-, -N(OH)- or -CO-V-, where V is a peptide having altogether 1 to 10 amino, imino and/or aza amino acids;
or in which R⁴, together with R⁸ and the atoms carrying them, form monocyclic or bicyclic, saturated or partially unsaturated ring systems having 5-12 ring members which, apart from carbon, can also contain 1 sulfur atom which can optionally be oxidized to the sulfoxide or sulfone;
b₃)
- a glycosyl radical, preferably a glucofuranosyl or glucopyranosyl radical, which is derived from naturally occurring aldotetroses, aldopentoses, aldohexoses, ketopentoses, ketohexoses, deoxyaldoses, aminoaldoses and oligosaccharides and their stereoisomers; or
b₄)
- an amino protective group;
R⁵ is
- hydrogen or
- (C₁-C₈)-alkyl, or
- together with R⁶ and the atoms carrying this radical, forms mono- or bicyclic, saturated or partially unsaturated ring systems having 5-12 ring members;
R⁶ is
- defined as R⁴ in b₁);
- hydroxyl or (C₁-C₄)-alkanoyloxy; or
- together with R⁷ and the atoms carrying this radical, forms cyclic, saturated or partially unsaturated ring systems, having 3 to 12 ring members; or
- together with R⁸ and the atoms carrying this, forms a mono- or bicyclic, saturated or partially unsaturated ring system having 5-12 ring members which, apart from carbon, can also contain 1 sulfur atom which can optionally be oxidized to the sulfoxide or sulfone; or can contain 1 nitrogen atom, where the ring system can optionally be substituted by amino;
R⁷ is
- hydrogen or
- (C₁-C₆)-alkyl;
R⁸ is
- hydrogen
- hydroxyl,
- (C₁-C₄)-alkanoyloxy or
- (C₁-C₈)-alkyl;
R⁹ and R¹⁰ are
- hydrogen,
- (C₁-C₈)-alkyl which can be substituted by
- amino,
- (C₁-C₄)-alkylamino,
- di-(C₁-C₄)-alkylamino,
- mercapto,
- carboxyl,
- hydroxyl or
- (C₁-C₄)-alkoxy,
- (C₃-C₇)-cycloalkyl,
- (C₁-C₄)-alkoxycarbonyl,
- (C₆-C₁₄)-aryl, (C₆-C₁₄)-aryl-(C₁-C₄)-alkoxycarbonyl which can be substituted in the aryl moiety as described in the case of R⁴,
- het or
- het-(C₁-C₄)-alkyl, where het is defined as described in the case of R⁴, or where
R⁹ and R¹⁰, together with the nitrogen atom carrying them, form monocyclic or bicyclic, saturated, partially unsaturated or aromatic ring systems which as ring members in addition to carbon also contain 1 or additionally 2 nitrogen atoms, 1 sulfur atom or 1 oxygen atom and can be substituted by (C₁-C₄)-alkyl, where in the above compounds of the formula I one or more amide groups (-CONH-) of the main chain can be replaced by -CH₂NR¹¹-, -CH₂S-, -CH₂O-, - OCH₂-, -CH₂CH₂-, -CH=CH-(cis and trans), -COCH₂-, -CH(OH)CH₂-, -CH₂SO-, -CH₂SO₂-, - COO-, -P(O)(OR¹²)CH₂- and -P(O)(OR¹²)NH-, or alternatively by an amide group of reverse polarity (-NHCO-);
in which R¹¹ and R¹² independently of one another are
- hydrogen or
- (C₁-C₄)-alkyl;
and their enantiomers and physiologically acceptable salts, which comprises treating homochiral α-aminoaldehydes of the formula VII 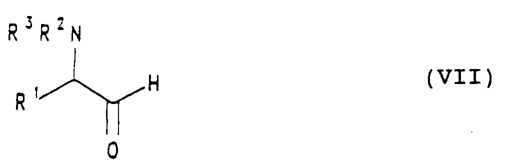 in which R¹, R² and R³ are defined as above,
with the NbCl₃-dimethoxyethane complex [NbCl₃ (DME)], simultaneous control over all four centers of chirality existing.

2. The process for the preparation of compounds of the formula I as claimed in claim 1, wherein
R¹ is a side-chain radical of the α-amino acids Gly, Ala, Val, Leu, Ile, Ser, Thr, Cys, Met, Pro, Lys, Arg, His, Asp, Asn, Glu, Gln, Phe, Tyr, Trp or Cha;
R² and R³ are identical or different and are
a)
- hydrogen
b)
- a radical of the formula II
in which o and p = 0,
n = 0 or 1 and
E is one of the abovementioned α-amino acids,
D is R⁴ or a radical of the formula III or IV, in which R⁴ is
b₁)
- hydrogen
- (C₁-C₉)-alkyl which is optionally monounsaturated or diunsaturated and which is optionally substituted by up to 3 identical or different radicals selected from the group comprising
- hydroxyl,
- (C₁-C₇)-alkoxy,
- carbamoyl,
- (C₁-C₈)-alkanoyloxy,
- (C₁-C₇)-alkoxycarbonyl,
- F or Cl,
- amino,
- (C₁-C₇)-alkylamino,
- di-(C₁-C₇)-alkylamino,
- (C₁-C₆)-alkoxycarbonylamino,
- (C₇-C₁₅)-aralkoxycarbonyl,
- (C₇-C₁₅)-aralkoxycarbonylamino,
- phenyl-(C₁-C₄)-alkoxy,
- 9-fluorenylmethoxycarbonylamino,
- (C₁-C₆)-alkylsulfonyl,
- (C₁-C₆)-alkylsulfinyl,
- (C₁-C₆)-alkylthio,
- (C₆-C₁₄)-aryl,
- (C₆-C₁₄)-aryl-(C₁-C₆)-alkyl or
- (C₆-C₁₄)-aryloxy-(C₁-C₆)-alkyl, in which the aryl moiety can in each case be optionally substituted by one, two or three identical or different radicals selected from the group consisting of the abovementioned preferred substituents of (C₁-C₉)-alkyl,
b₂)
- a radical of the formula VI, in which
R^{4a} is defined as R⁴ in b₁) and W is -CO-, -O-CO-, -SO₂-, -SO-, -S-, -NHCO-, -CH(OH)-;
b₄)
- an amino protective group Fmoc, Z or Boc,
R⁵ and R⁷ are hydrogen,
R⁶ is
- defined as R⁴, and
R⁸ is
- hydrogen,
- hydroxyl,
- (C₁-C₄)-alkanoyloxy or
- (C₁-C₈)-alkyl.

3. The process for the preparation of compounds of the formula I as claimed in one of claims 1 or 2, wherein one of the radicals R² or R³ is hydrogen.

4. The process for the preparation of compounds of the formula I as claimed in one of claims 1 to 3, wherein the compounds of the formula I have the SRRS-configuration or RSSR-configuration.

5. The process for the preparation of compounds of the formula I as claimed in one of claims 1 to 4, wherein
R¹ is
- a side-chain radical of the α-amino acids Ala, Val, Leu, Ile, Pro, Phe, Cha or Tyr,
R² and R³ are identical or different and are
a)
- hydrogen
b)
- a radical of the formula II in which
o and p = 0,
n is 0 or 1 and
E is Ala, Val, Leu, Ile, Pro, Phe, Cha or Tyr;
D is R⁴ or a radical of the formula IV where R⁴ is
b₁)
- hydrogen,
- (C₁-C₄)-alkyl,
- phenyl or naphthyl,
- phenylmethyl or naphthylmethyl,
b₂)
- a radical of the formula VI in which R^{4a} is defined as R⁴ in b₁) and W is -CO-, -O-CO-, -SO₂-, -SO-, -S-, -NHCO-, -CH(OH)-, or
b₄)
- an amino protective group Fmoc, Z or Boc,
R⁵, R⁷ and R⁸ are hydrogen, and
R⁶ is defined as R⁴ in b₁).

6. The process for the preparation of compounds of the formula I as claimed in one of claims 1 to 5, wherein, for the preparation of a compound of the formula I in the SRRS-configuration, a compound of the formula VII having the S-configuration is reacted at a temperature between room temperature and the boiling point of the reaction mixture.

7. The process for the preparation of compounds of the formula I as claimed in one of claims 1 to 6, wherein a compound of the formula VII is treated with the NbCl₃-dimethoxyethane complex in the presence of THF as solvent.

## Revendications

1. Procédé pour la préparation de composés de formule I 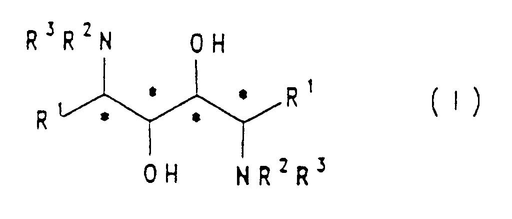 dans laquelle
R¹ représente un reste de chaîne latérale d'un α-aminoacide naturel ou non naturel;
R² et R³ sont identiques ou différents et représentent
a) un atome d'hydrogène,
b) un radical de formule
D-(E)ₙ-(F)ₒ-(G)ₚ (II)
E, F et G représentant, indépendamment les uns des autres, un aminoacide, azaaminoacide ou iminoacide naturel ou non naturel;
n, o, p représentant, indépendamment les uns des autres, 0 ou 1;
D représentant R⁴ ou un radical de formule III, IV ou V 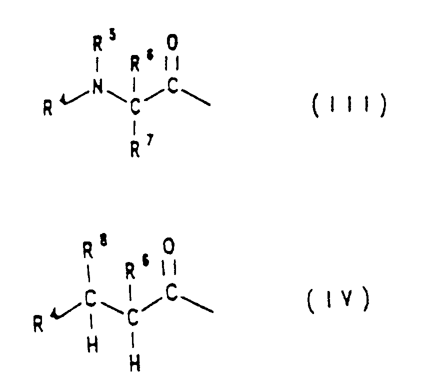 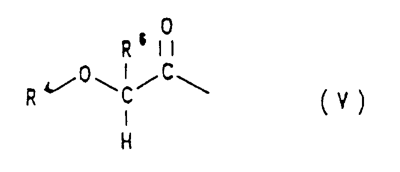 formules dans lesquelles R⁴ représente
b₁)
- un atome d'hydrogène,
- le groupe carboxy,
- un groupe alkyle en C₁-C₁₈ qui est éventuellement 1 ou 2 fois insaturé et qui peut éventuellement être substitué par jusqu'à 3 radicaux, identiques ou différents, choisis parmi les groupes
- mercapto,
- hydroxy
- alcoxy en C₁-C₇,
- carbamoyle,
- alcanoyloxy en C₁-C₈,
- carboxy,
- alcoxy(C₁-C₇)carbonyle,
- F, Cl, Br, I,
- amino,
- amidino qui peut éventuellement être substitué par 1, 2 ou 3 radicaux alkyle en C₁-C₈,
- guanidino qui peut éventuellement être substitué par 1 ou 2 radicaux benzyloxycarbonyle ou par 1, 2, 3 ou 4 radicaux alkyle en C₁-C₈,
- alkyl(C₁-C₇)amino,
- dialkyl(C₁-C₇)amino,
- alcoxy(C₁-C₆)carbonylamino,
- aralcoxy(C₇-C₁₅)carbonyle,
- aralcoxy(C₇-C₁₅)carbonylamino,
- phényl-alcoxy(C₁-C₄),
- 9-fluorénylméthoxycarbonylamino,
- alkyl(C₁-C₆)sulfonyle,
- alkyl(C₁-C₆)sulfinyle,
- alkyl(C₁-C₆)thio,
- hydroxamino,
- hydroximino,
- sulfamoyle,
- sulfo,
- carboxamido,
- formyle,
- hydrazono,
- imino,
- un radical CONR⁹R¹⁰,
- par jusqu'à 6 groupes hydroxy ou
- par jusqu'à 5 groupes alcanoyloxy en C₁-C₈;
- cycloalkyle en C₃-C₁₈ mono-, bi- ou tricyclique,
- cycloalkyl(C₃-C₁₈)-alkyle(C₁-C₆),
le fragment cycloalkyle étant dans chaque cas éventuellement substitué par 1 ou 2 radicaux, identiques ou différents, choisis parmi
- F, Cl, Br, I,
- carboxy,
- carbamoyle,
- carboxyméthoxy,
- hydroxy,
- alcoxy en C₁-C₇,
- alkyle en C₁-C₇,
- alkyloxy(C₁-C₇)carbonyle,
- amino,
- alkyl(C₁-C₆)amino-alkyle(C₁-C₆),
- dialkyl(C₁-C₆)amino-alkyle(C₁-C₆),
- amidino,
- hydroxamino,
- hydroximino,
- hydrazono,
- imino,
- guanidino,
- alcoxy(C₁-C₆)sulfonyle,
- alcoxy(C₁-C₆)sulfinyle,
- alcoxy(C₁-C₆)carbonylamino,
- aryl(C₆-C₁₂)-alcoxy(C₁-C₄)carbonylamino,
- alkyl(C₁-C₇)amino,
- dialkyl(C₁-C₇)amino et
- trifluorométhyle;
- aryle en C₆-C₁₄,
- aryl(C₆-C₁₄)-alkyle(C₁-C₆),
- aryloxy(C₆-C₁₄)-alkyle(C₁-C₆) ou
- aryl(C₆-C₁₄)-cycloalkyle(C₃-C₈),
le fragment aryle étant éventuellement substitué par 1, 2 ou 3 radicaux, identiques ou différents, choisis parmi
- F, Cl, Br, I,
- hydroxy,
- mono-, di- ou trihydroxyalkyle en C₁-C₄,
- trifluorométhyle,
- formyle,
- carboxamido,
- mono- ou dialkyl(C₁-C₄)aminocarbonyle,
- nitro,
- alcoxy en C₁-C₇,
- alkyle en C₁-C₇,
- alcoxy(C₁-C₇)carbonyle,
- amino,
- alkyl(C₁-C₇)amino,
- dialkyl(C₁-C₇)amino,
- carboxy,
- carboxyméthoxy,
- aminoalkyle(C₁-C₇),
- alkyl(C₁-C₇)amino-alkyle(C₁-C₇),
- dialkyl(C₁-C₇)amino-alkyle(C₁-C₇),
- alcoxy(C₁-C₇)carbonylméthoxy,
- carbamoyle,
- sulfamoyle,
- alcoxy(C₁-C₇)sulfonyle,
- alkyl(C₁-C₈)sulfonyle,
- sulfoalkyle(C₁-C₈),
- guanidinoalkyle(C₁-C₈) et
- alcoxy(C₁-C₆)carbonylamino;
- Het,
- Het-alkyle(C₁-C₆),
- Het-cycloalkyle(C₃-C₈),
- Het-cycloalkyl(C₃-C₈)-alkyle(C₁-C₄),
- Het-cycloalcoxy(C₃-C₈)-alkyle(C₁-C₄),
- Het-thioalkyle(C₁-C₆),
- Het-thiocycloalkyle(C₃-C₈),
- Het-thiocycloalkyl(C₃-C₈)-alkyle(C₁-C₄),
Het représentant dans chaque cas le reste d'un système cyclique monocyclique à 5-7 chaînons ou bicyclique à 8-10 chaînons, qui peut être aromatique, partiellement ou totalement hydrogéné ou fusionné à un noyau benzénique, et qui peut contenir comme hétéroéléments 1, 2, 3 ou 4 atomes ou groupes différents choisis parmi N, O, S, NO, SO, SO₂, qui peut être substitué par 1 à 6 groupes hydroxy et qui est éventuellement mono-, di- ou trisubstitué comme défini en b₁) pour le groupe aryle en C₆-C₁₄ et/ou par le groupe oxo, ou représente un radical NR⁹R¹⁰, ou
b₂)
- un radical de formule VI
R^{4a}-W (VI)
dans laquelle R^{4a} est défini comme R⁴ en b₁), et W représente -CO-, -CS-, -O-CO-, -SO₂-, -SO-, -S-, -NHSO₂-, -NHCO-, -CH(OH)-, -N(OH)- ou -CO-V-, V représentant un peptide comportant au total 1 à 10 amino-, imino- et/ou azaaminoacides;
ou dans laquelle R⁴ forme, conjointement avec R⁸ et les atomes qui les portent, des systèmes cycliques mono- ou bicycliques, saturés ou partiellement insaturés, comportant 5-12 chaînons formant le(s) cycle(s), qui peuvent contenir, en plus d'atomes de carbone, encore un atome de soufre qui peut éventuellement être oxydé en sulfoxyde ou en sulfone;
b₃)
- un radical glycosyle, de préférence un radical glucofurannosyle ou glucopyrannosyle, qui dérive d'aldotétroses, aldopentoses, aldohexoses, cétopentoses, cétohexoses, désoxyaldoses, aminoaldoses et oligosaccharides existant dans la nature, ainsi que de leurs stéréoisomères; ou
b₄)
- un groupe protecteur de fonction amino;
R⁵ représente
- un atome d'hydrogène ou
- un groupe alkyle en C₁-C₈, ou
- conjointement avec R⁶ et les atomes qui les portent, des systèmes cycliques saturés ou partiellement insaturés, ayant de 5 à 12 chaînons formant le cycle;
R⁶
- est défini comme R⁴ en b₁);
- représente un groupe hydroxy ou alcanoyloxy en C₁-C₄; ou
- forme, conjointement avec R⁷ et l'atome qui les porte, des systèmes cycliques saturés ou partiellement insaturés, ayant de 3 à 12 chaînons formant le cycle; ou
- conjointement avec R⁸ et les atomes qui le portent, un système cyclique mono- ou bicyclique, saturé ou partiellement insaturé, comportant 5-12 chaînons formant le(s) cycle(s), qui peut contenir, en plus d'atomes de carbone, encore un atome de soufre qui peut éventuellement être oxydé en sulfoxyde ou sulfone; ou peut contenir un atome d'azote, le système cyclique pouvant éventuellement être substitué par le groupe amino;
R⁷ représente
- un atome d'hydrogène ou
- un groupe alkyle en C₁-C₆;
R⁸ représente
- un atome d'hydrogène,
- le groupe hydroxy,
- un groupe alcanoyloxy en C₁-C₄ ou
- un groupe alkyle en C₁-C₈;
R⁹ et R¹⁰ représentent
- un atome d'hydrogène,
- un groupe alkyle en C₁-C₈ qui peut être substitué par un groupe
- amino,
- alkyl(C₁-C₄)amino,
- dialkyl(C₁-C₄)amino,
- mercapto,
- carboxy,
- hydroxy ou
- alcoxy en C₁-C₄,
- cycloalkyle en C₃-C₇,
- alcoxy(C₁-C₄)carbonyle,
- aryle(C₆-C₁₄), aryl(C₆-C₁₄)-alcoxy(C₁-C₄)-carbonyle, qui peuvent être substitués sur le fragment aryle comme décrit pour R⁴,
- Het ou
- Het-alkyle(C₁-C₄), Het étant défini comme décrit pour R⁴, ou
R⁹ et R¹⁰ forment ensemble, avec l'atome d'azote qui les porte, des systèmes cycliques monocycliques ou bicycliques, saturés, partiellement insaturés ou aromatiques, qui peuvent contenir en tant que chaînons formant le(s) cycle(s), en plus d'atomes de carbone, encore 1 ou 2 autres atomes d'azote, 1 atome de soufre ou 1 atome d'oxygène, et être substitués par des groupes alkyle en C₁-C₄, un ou plusieurs groupes amido (-CONH-) de la chaîne principale, dans les composés précédents de formule I, pouvant être remplacés par -CH₂NR¹¹-, -CH₂S-, -CH₂O-, -OCH₂-, -CH₂CH₂-, -CH=CH-(*cis* et *trans*), -COCH₂-, -CH(OH)CH₂-, -CH₂SO-, -CH₂SO₂-, -COO-, -P(O)(OR¹²)CH₂- et -P(O)(OR¹²)NH-, ou également par un groupe amido à polarité inversée (-NHCO-);
groupes dans lesquels R¹¹ et R¹² représentent, indépendamment l'un de l'autre,
- un atome d'hydrogène ou
- un groupe alkyle en C₁-C₄;
ainsi que de leurs énantiomères et sels physiologiquement acceptables,
caractérisé en ce que l'on traite des α-aminoaldéhydes homochiraux de formule VII 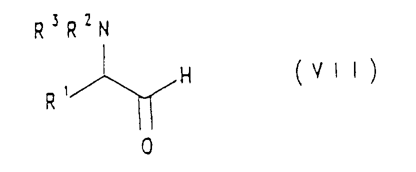 dans laquelle R¹, R² et R³ sont tels que définis plus haut,
par le complexe NbCl₃-diméthoxyéthane [NbCl₃ (DME)], les quatre centres de chiralité étant tous simultanément sous contrôle.

2. Procédé pour la préparation de composés de formule I selon la revendication 1, caractérisé en ce que
R¹ représente un reste de chaîne latérale des α-aminoacides Gly, Ala, Val, Leu, Ile, Ser, Thr, Cys, Met, Pro, Lys, Arg, His, Asp, Asn, Glu, Gln, Phe, Tyr, Trp ou Cha;
R² et R³ sont identiques ou différents et représentent
a)
- un atome d'hydrogène,
b)
- un radical de formule II
dans lequel o et p = 0,
n = 0 ou 1 et
E représente l'un des α-aminoacides indiqués plus haut,
D représente R⁴ ou un radical de formule III ou IV, dans lequel R⁴ représente
b₁)
- un atome d'hydrogène,
- un groupe alkyle en C₁-C₉ qui est éventuellement une ou deux fois insaturé et qui est éventuellement substitué par jusqu'à 3 radicaux identiques ou différents, choisis parmi les radicaux
- hydroxy,
- alcoxy en C₁-C₇,
- carbamoyle,
- alcanoyloxy en C₁-C₈,
- alcoxy(C₁-C₇)carbonyle,
- F, Cl,
- amino,
- alkyl(C₁-C₇)amino,
- dialkyl(C₁-C₇)amino,
- alcoxy(C₁-C₆)carbonylamino,
- aralcoxy(C₇-C₁₅)carbonyle,
- aralcoxy(C₇-C₁₅)carbonylamino,
- phénylalcoxy(C₁-C₄),
- 9-fluorénylméthoxycarbonylamino,
- alkyl(C₁-C₆)sulfonyle,
- alkyl(C₁-C₆)sulfinyle,
- alkyl(C₁-C₆)thio,
- aryle en C₆-C₁₄,
- aryl(C₆-C₁₄)-alkyle(C₁-C₆) ou
- aryloxy(C₆-C₁₄)-alkyle(C₁-C₆), le fragment aryle pouvant dans chaque cas être éventuellement substitué par 1, 2 ou 3 radicaux, identiques ou différents, choisis dans le groupe constitué par les substituants préférés du groupe alkyle en C₁-C₉, indiqués plus haut,
b₂)
- un radical de formule VI dans lequel R^{4a} est défini comme R⁴ en b₁), et W représente -CO-, -O-CO-, -SO₂-, -SO-, -S-, -NHCO-, -CH(OH)-;
b₄)
- un groupe protecteur Fmoc, Z ou Boc de fonction amino,
R⁵ et R⁷ représentent des atomes d'hydrogène,
R⁶ est défini comme R⁴, et
R⁸ représente
- un atome d'hydrogène,
- le groupe hydroxy,
- un groupe alcanoyloxy en C₁-C₄ ou
- un groupe alkyle en C₁-C₈.

3. Procédé pour la préparation de composés de formule I selon la revendication 1 ou 2, caractérisé en ce que l'un des radicaux R² et R³ représente un atome d'hydrogène.

4. Procédé pour la préparation de composés de formule I selon l'une des revendications 1 à 3, caractérisé en ce que les composés de formule I ont une configuration SRRS ou une configuration RSSR.

5. Procédé pour la préparation de composés de formule I selon l'une des revendications 1 à 4, caractérisé en ce que
R¹ représente un reste de chaîne latérale des α-aminoacides Ala, Val, Leu, Ile, Pro, Phe, Cha ou Tyr,
R² et R³ sont identiques ou différents et représentent
a)
- un atome d'hydrogène,
b)
- un radical de formule II dans lequel
o et p = 0,
n est 0 ou 1 et
E représente Ala, Val, Leu, Ile, Pro, Phe, Cha ou Tyr;
D représente R⁴ ou un radical de formule IV dans laquelle R⁴ représente
b₁)
- un atome d'hydrogène,
- un groupe alkyle en C₁-C₄,
- phényle ou naphtyle,
- phénylméthyle ou naphtylméthyle,
b₂)
- un radical de formule VI dans lequel R^{4a} est défini comme R⁴ en b₁) et W représente - CO-, -O-CO-, -SO₂-, -SO-, -S-, -NHCO-, -CH(OH)-, ou
b₄)
- un groupe protecteur Fmoc, Z ou Boc de fonction amino,
R⁵, R⁷ et R⁸ représentent des atomes d'hydrogène et
R⁶ est défini comme R⁴ en b₁).

6. Procédé pour la préparation de composés de formule I selon l'une des revendications 1 à 5, caractérisé en ce que, pour la préparation d'un composé de formule I en configuration SRRS, on fait réagir un composé de formule VII à configuration S, à une température comprise entre la température ambiante et la température d'ébullition du mélange réactionnel.

7. Procédé pour la préparation de composés de formule I selon l'une des revendications 1 à 6, caractérisé en ce que l'on traite un composé de formule VII, en présence de THF en tant que solvant, par le complexe NbCl₃-diméthoxyéthane.
